(19) Europäisches Patentamt
European Patent Office
Office européen des brevets

(11) EP 2 892 914 B1

(12) EUROPEAN PATENT SPECIFICATION

(45) Date of publication and mention of the grant of the patent:
**23.05.2018 Bulletin 2018/21**

(21) Application number: **13834677.0**

(22) Date of filing: **09.09.2013**

(51) Int Cl.:
***C07K 14/00*** (2006.01) ***C07K 7/00*** (2006.01)
***A61K 38/04*** (2006.01) ***A61K 38/16*** (2006.01)
***A61L 12/14*** (2006.01) ***A61P 31/04*** (2006.01)

(86) International application number:
**PCT/SG2013/000390**

(87) International publication number:
**WO 2014/039013 (13.03.2014 Gazette 2014/11)**

(54) **TETRABRANCHED PEPTIDES AND ANALOGUES AS A NEW CLASS OF ANTIMICROBIALS AND THEIR PREPARATION THEREOF**

TETRAVERZWEIGTE PEPTIDE UND ANALOGA ALS NEUE KLASSE VON ANTIMIKROBIELLEN WIRKSTOFFEN UND DEREN HERSTELLUNG

PEPTIDES TÉTRA-RAMIFIÉS ET ANALOGUES EN TANT QUE NOUVELLE CLASSE D’ANTIMICROBIENS, ET LEUR PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.09.2012 SG 201206715**

(43) Date of publication of application:
**15.07.2015 Bulletin 2015/29**

(73) Proprietors:
- **Agency for Science, Technology and Research**
  **Singapore 138632 (SG)**
- **Singapore Health Services Pte Ltd**
  **Singapore 168753 (SG)**

(72) Inventors:
- **BEUERMAN, Roger**
  **Singapore 168751 (SG)**
- **LIU, Shouping**
  **Singapore 168751 (SG)**
- **RAJAMANI, Lakshminarayanan**
  **Singapore 168751 (SG)**
- **VERMA, Chandra**
  **Singapore 138671 (SG)**
- **LI, Jianguo**
  **Singapore 168751 (SG)**

(74) Representative: **Brann AB**
**P.O. Box 3690**
**Drottninggatan 27**
**103 59 Stockholm (SE)**

(56) References cited:
**WO-A1-2009/131548**

- **ZHOU L ET AL: "The structural parameters for antimicrobial activity, human epithelial cell cytotoxicity and killing mechanism of synthetic monomer and dimer analogues derived from hBD3 C-terminal region", AMINO ACIDS ; THE FORUM FOR AMINO ACID AND PROTEIN RESEARCH, SPRINGER-VERLAG, VI, vol. 40, no. 1, 17 April 2010 (2010-04-17) , pages 123-133, XP019870157, ISSN: 1438-2199, DOI: 10.1007/S00726-010-0565-8**
- **LIU S P ET AL: "Multivalent Antimicrobial Peptides as Therapeutics: Design Principles and Structural Diversities", INTERNATIONAL JOURNAL OF PEPTIDE RESEARCH AND THERAPEUTICS ; FORMERLY KNOWN AS LETTERS IN PEPTIDE SCIENCE, KLUWER ACADEMIC PUBLISHERS, DO, vol. 16, no. 3, 26 August 2010 (2010-08-26), pages 199-213, XP019825531, ISSN: 1573-3904**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 892 914 B1

- XIAO WEI TAN ET AL: "Dual functionalization of titanium with vascular endothelial growth factor and [beta]-defensin analog for potential application in keratoprosthesis", JOURNAL OF BIOMEDICAL MATERIALS RESEARCH. PART B: APPLIED BIOMATERIALS, vol. 100B, no. 8, 23 July 2012 (2012-07-23), pages 2090-2100, XP055257862, US ISSN: 1552-4973, DOI: 10.1002/jbm.b.32774
- Y. BAI ET AL: "Progressive Structuring of a Branched Antimicrobial Peptide on the Path to the Inner Membrane Target", JOURNAL OF BIOLOGICAL CHEMISTRY, vol. 287, no. 32, 3 August 2012 (2012-08-03), pages 26606-26617, XP055257857, US ISSN: 0021-9258, DOI: 10.1074/jbc.M112.363259
- Siti Safie: "Optimizing antifungal properties of tetrabranched peptide B4010.", Thesis, 18 May 2012 (2012-05-18), XP055258021, Retrieved from the Internet: URL:https://repository.ntu.edu.sg/bitstream/handle/10356/49358/Final Thesis.pdf?sequence=1&isAllowed=n [retrieved on 2016-03-14]

## Description

Field of the invention

The present invention relates to tetrameric forms of peptides, with antimicrobial properties, particularly antifungal properties. The invention also relates to methods of making these tetrameric peptides. The invention also relates to the use of these multimers for inhibiting the growth of microorganisms, particularly fungus. The invention further relates to composition comprising these tetrameric peptides.

Background of the invention

Antifungals are used to inhibit the growth of fungus and/or kill fungus (fungicide). For example, antifungal medications are drugs are used to treat infections caused by fungus and to prevent the development of fungal infections in patients, especially those with weakened immune systems. Pathogenic fungal infections are the seventh most common cause of infection-related deaths in the US. In fact, recent surveys suggest that fungal diseases are posing a growing threat to the global biota.

In comparison to antibacterial drugs, the number of available antifungals is limited since common drug targets in fungi are homologues of similar molecular types in human which might also be inhibitied. Examples of antifungals include polyenes, azoles, allylamines, fluocytosine and echniocandins (Figure 9). Moreover, recent reports indicate increasing incidence of fungal resistance to existing antifungals.

Fungal infection of the eye, for example, fungal keratitis or keratomycosis, is common in tropical climates. In advanced countries, use of contact lens or lens care solution is the major risk factor for fungal keratitis. Ever since the first report on contact lens-associated fungal keratitis, several countries documented the incidence of ocular fungal pathogens infection (Figure 10). For fungal keratitis, natamycin is the only US FDA approved ophthalmic antifungal.

Zhou et al. (2011) Amino Acids 40: 123-133 describes the synthesis, antibiotic activity and cytotoxicity of sequence derivatives of hBD3 and a V2-dimer.

Liu et al. (2010) Int. J. Pept. Res. Ther. 16: 199-213 is a review discussing natural and synthetic multivalent antimicrobial peptides.

Tan et al. (2012) J. Biomed. Mat. Res. B 100B, 2090-2100 describes the functionalization of a titanium alloy substrate with recombinant vascular endothelial growth factor and anti-microbial peptide, SESB2V.

Safie (2012) Thesis entitled "Optimizing antifungal properties of tetrabranched peptide B4010" describes the tetrameric peptide B410, and the use of alanine scanning to search for derivatives.

WO2009/131548 A1 describes the development of multimers of the peptide hBD3, and discloses the structure and synthesis of a V2 tetratmer.

Bai et al. (2012) discloses a branched antimicrobial peptide for targeting Gram-negative bacteria.

The emergence of drug-resistance in fungus combined with an increased number of immune-compromised patients has limited the therapeutic options for clinicains and underscores the need for new classes of antifungals.

It is therefore desirable to develop antimicrobials particularly suited as antifungals, which are broad spectrum, suitable for many uses, especially for the treatment of ocular infections and preferably, resilient to the development of fungus resistance.

Summary of the invention

According to a first aspect, the present invention relates to an isolated peptide tetramer selected from the group consisting of $[(RGAAVVRR)_2K]_2KK_i$, $[(RGRKVVAA)_2K]_2KK_i$, $[(RGAKAVRR)_2K]_2KK_i$, $[(RGAAAVRR)_2K]_2KK_i$, $[(RGAKAARR)_2K]_2KK_i$, $[(RGRAAARR)_2K]_2KK_i$, $[(RGAAAARR)_2K]_2KK_i$, $[(RGRKAAAA)_2K]_2KK_i$; wherein i = 0 or 1.

Embodiments of the isolated peptide tetramer are defined by the dependent claims.

The invention also relates to uses thereof of the isolated peptide tetramer derivative or isolated peptide tetramer as described herein.

The invention also includes a method of preparing the isolated peptide derivative or isolated peptide tetramer as described herein.

Brief description of the figures

Figure 1: Structure of monomer, linear retrodimer, peptide dimer B2088, peptide tetramer B4010, scrambled B4010 and natamycin.

Figure 2: Kinetics of B4010 mediated killing of *C. albicans* (A) ATCC 10231 and (B) clinical isolate DF2672R. 2x$10^6$ cells were incubated with indicated concentration of B4010, amphotericin B and natamycin. At the indicated time points aliquots of the samples were plated on SDA and the cell viability was determined.

Figure 3: Antifungal properties of B4010 in the presence of metal ions and complex biological fluids. (A) MIC values determined in the presence of monovalent and divalent metal ions. The numbers above the bars indicate the determined MIC values. (B) Candidacidal properties of B4010 in the presence of metal ions. The concentration of B4010 was 5.5 μM. (C) Antifungal activities in the presence of trypsin (Trypsin:B4010 = 1:100). (D) Candidacidal activity of B4010 in the presence of 50% rabbit tear fluid.

Figure 4: Toxicity and corneal repithelialization rate of B4010. (A) Haemolytic activity of B4010 and polyene antifungals (natamycin and amphotericin B) against 4% rabbit erythrocytes. (B) Cytotoxicity of B4010 and the polyene antifungals against human corneal epithelial (HCE) cells. (C) Effect of B4010 on corneal reepitheliazation of New Zealand white rabbits as measured by fluorescein staining. The peptide concentration was 22μM.

Figure 5: Effect of B4010 on cytoplasmic membrane potential, membrane permeabilization and morphology of *C. albicans.* (A) SDS-PAGE showing lack of affinity of B4010 for fungal cell wall polysaccharide, β-chitin. (B) SYTOX Green uptake of *C. albicans* induced by varying concentration of B4010. (C) B4010-mediated membrane depolarization monitored by diSC35 assay. (D) B4010-induced extracellular ATP release of *C. albicans.* The inset shows the kinetics of ATP release. B4010 concentration was 5.5 μM. (E) SEM of untreated *C. albicans.* Scale bar is 2 μm (inset scale bar = 200 nm). (F) SEM image of C. albicans treated with 5.5 μM B4010. Scale bar is 1 μm (inset scale bar = 100 nm).

Figure 6: Effects of various additives on candidacidal and membrane permeabilizing properties of B4010. (A) Effect of CCCP and $NaN_3$ on membrane potential. (B) Effect of various additives on (B) viability and (C) ATP release. (D) Effect of ion-channel inhibitors on membrane potential. The arrows indicate the time of addition of additives

Figure 7: Interaction of B4010 with mammalian and fungal model membrane. (A) Time course of calcein release from SUVs of PC:PE:PS:ergosterol after the addition of B4010 and Scrambled B4010. (B) Time course of calcein release from PC:cholesterol SUVs. The peptide:lipid ratio is indicated in the graphs.

Figure 8: CD spectra of B4010 and Scrambled B4010 in PBS (pH = 7.0)

Figure 9: Examples of current antifungals.

Figure 10: Survey of ocular fungal pathogens from 2007-2012

## Definitions

Non-proteogenic amino acid refers to an amino acid which is not normally found in naturally occurring proteins which are typically made up of different numbers and combinations of 20 standard amino acids in their sequences.

Protected amino acid means an amino acid with a protecting group linked to either the carboxy group or the amine group protecting that group to allow the formation of a peptide bond with only the carboxy group or the amine group but not both. If the amine group is protected, only the carboxy group is available to form a peptide bond with another amine group. If the carboxy group is protected, only the amine group is available to form a peptide bond with another carboxy group. The terminal amine group or terminal carboxy group of a polypeptide may similarly be protected so that a peptide bond may be formed from the carboxy or amine end only.

## Detailed description of the invention

The present invention relates to an isolated peptide tetramer derivative of peptide tetramer $[(RGRKVVRR)_2K]_2KK_i$ with an initial peptide monomer (RGRKWRR); comprising two or more alanine substitutions, compared to the initial peptide monomer; wherein i = 0 or 1. The sequence of the initial peptide monomer is RGRKWRR (SEQ ID NO: 1).

If i = 0, an isolated peptide tetramer has the structure $[(RGRKWRR)_2K]_2K$.
If i = 1, an isolated peptide tetramer has the structure $[(RGRKWRR)_2K]_2KK$.

The isolated peptide tetramer derivative has the formula $[(peptide)_2K]_2KK$.

In particular, the isolated peptide tetramer derivative is branched with the structure:

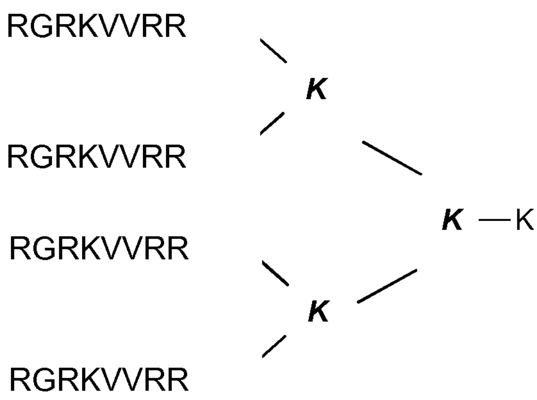

The isolated peptide tetramer derivative is similarly branched with the structure:

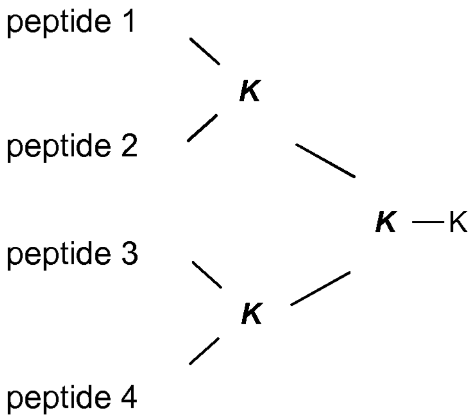

Each of peptide 1, peptide 2, peptide 3 and peptide 4 is derived from the initial peptide monomer as described. Peptide 1, peptide 2, peptide 3 and peptide 4 have the same sequence.

Peptide 1, peptide 2, peptide 3 and peptide 4 may consist of SEQ ID NOs: 10-12 or 14-18.

The amino acid substitution comprises successively substituting two or more amino acids of the initial peptide monomer (RGRKWRR) with alanine.

In embodiments not related to the present invention, an isolated peptide tetramer derivative may be selected from the group consisting of $[(AGRKVVRR)_2K]_2KK_i$, $[(RARKVVRR)_2K]_2KK_i$, $[(RGAKVVRR)_2K]_2KK_i$, $[(RGRAVVRR)_2K]_2KK_i$, $[(RGRKAVRR)_2K]_2KK_i$, $[(RGRKVARR)_2K]_2KK_i$, $[(RGRKVVAR)_2K]_2KK_i$ and $[(RGRKVVRA)_2K]_2KK_i$; wherein i = 0 or 1.

AGRKVVRR = SEQ ID NO: 2

RARKVVRR = SEQ ID NO: 3

RGAKVVRR = SEQ ID NO: 4

RGRAWRR = SEQ ID NO: 5

RGRKVARR = SEQ ID NO: 6

RGRKVARR = SEQ ID NO: 7

RGRKWAR = SEQ ID NO: 8

RGRKVVRA = SEQ ID NO: 9

When i = 0, the isolated peptide tetramer derivative is selected from the group consisting of $[(AGRKVVRR)_2K]_2K$, [(RAR-

KVVRR)$_2$K]$_2$K, [(RGAKVVRR)$_2$K]$_2$K, [(RGRAVVRR)$_2$K]$_2$K, [(RGRKAVRR)$_2$K]$_2$K, [(RGRKVARR)$_2$K]$_2$K, [(RGRKVVAR)$_2$K]$_2$K and [(RGRKVVRA)$_2$K]$_2$K

When i = 1,the isolated peptide tetramer derivative is selected from the group consisting of [(AGRKWRR)$_2$K]$_2$K, [(RARKVVRR)$_2$K]$_2$KK, [(RGAKVVRR)$_2$K]$_2$KK, [(RGRAVVRR)$_2$K]$_2$KK, [(RGRKAVRR)$_2$K]$_2$KK, [(RGRKVARR)$_2$K]$_2$KK, [(RGRKVVAR)$_2$K]$_2$KK and [(RGRKVVRA)$_2$K]$_2$KK.

In embodiments of the invention, two or more of the amino acids of the initial peptide monomer are substituted with alanine residues.

For example, the isolated peptide tetramer derivative may be selected from the group consisting of [(RGAAVVRR)$_2$K]$_2$KK$_i$, [(RGRKVVAA)$_2$K]$_2$KK$_i$, [(RGAKAVRR)$_2$K]$_2$KK$_i$, [(RGAAAVRR)$_2$K]$_2$KK$_i$, [(RGAKAARR)$_2$K]$_2$KK$_i$, [(RGRAAARR)$_2$K]$_2$KK$_i$, [(RGAAAARR)$_2$K]$_2$KK$_i$, [(RGRKAAAA)$_2$K]$_2$KK$_i$; wherein i = 0 or 1.

RGAAVVRR = SEQ ID NO: 10

RGRKVVAA = SEQ ID NO: 11

RGAKAVRR = SEQ ID NO: 12

RGRKAARR = SEQ ID NO: 13

RGAAAVRR = SEQ ID NO: 14

RGAKAARR = SEQ ID NO: 15

RGRAAARR = SEQ ID NO: 16

RGAAAARR = SEQ ID NO: 17

RGRKAAAA = SEQ ID NO: 18

When i = 0, the isolated peptide tetramer derivative is selected from the group consisting of [(RGAAVVRR)$_2$K]$_2$K, [(RGRKVVAA)$_2$K]$_2$K, [(RGAKAVRR)$_2$K]$_2$KK$_i$, [(RGAAAVRR)$_2$K]$_2$K, [(RGAKAARR)$_2$K]$_2$K$_i$, [(RGRAAARR)$_2$K]$_2$K, [(RGAAAARR)$_2$K]$_2$K, [(RGRKAAAA)$_2$K]$_2$K.

When i = 1, the isolated peptide tetramer derivative is selected from the group consisting of [(RGAAVVRR)$_2$K]$_2$KK, [(RGRKVVAA)$_2$K]$_2$KK, [(RGAKAVRR)$_2$K]$_2$KK, [(RGAAAVRR)$_2$K]$_2$KK, [(RGAKAARR)$_2$K]$_2$KK, [(RGRAAARR)$_2$K]$_2$KK, [(RGAAAARR)$_2$K]$_2$KK, [(RGRKAAAA)$_2$K]$_2$KK.

In a further embodiment not related to the invention, an isolated peptide tetramer derivative may comprise [VRGRVRKR)$_2$K]$_2$KK$_i$; wherein i = 0 or 1. In this embodiment, there is a rearrangement of the initial peptide monomer (RGRKWRR); i.e. the sequence of the initial peptide monomer is scrambled. When i = 0, the isolated peptide tetramer derivative is [VRGRVRKR)$_2$K]$_2$K. When i = 1, the isolated peptide tetramer derivative is [VRGRVRKR)$_2$K]$_2$KK

VRGRVRKR = SEQ ID NO: 19

In another example not related to the invention, the amino acid deletion may comprise successively excluding an amino acid from the N-terminus of at least one initial peptide monomer. Accordingly, the isolated peptide tetramer derivative may be selected from the group consisting of [(GRKVVRR)$_2$K]$_2$KK$_i$, [(RKVVRR)$_2$K]$_2$KK$_i$, [(KVVRR)$_2$K]$_2$KK$_i$, [(VVRR)$_2$K]$_2$KK$_i$, [(VRR)$_2$K]$_2$KK$_i$, [(RR)$_2$K]$_2$KK$_i$ and [(R)$_2$K]$_2$KK$_i$; wherein i = 0 or 1.

GRKVVRR = SEQ ID NO: 20

RKVVRR = SEQ ID NO: 21

KVVRR = SEQ ID NO: 22

WRR = SEQ ID NO: 23

VRR = SEQ ID NO: 24

RR = SEQ ID NO: 25

R = SEQ ID NO: 26

When i = 0, the isolated peptide tetramer derivative is selected from the group consisting of $[(GRKVVRR)_2K]_2K$, $[(RKVVRR)_2K]_2K$, $[(KVVRR)_2K]_2K$, $[(VVRR)_2K]_2K$, $[(VRR)_2K]_2K$, $[(RR)_2K]_2K$ and $[(R)_2K]_2K$.

When i = 1, the isolated peptide derivative is selected from the group consisting of $[(GRKVVRR)_2K]_2KK$, $[(RKVVRR)_2K]_2KK$, $[(KVVRR)_2K]_2KK$, $[(VVRR)_2K]_2KK$, $[(VRR)_2K]_2KK$, $[(RR)_2K]_2KK$ and $[(R)_2K]_2KK$.

In a further embodiment not related to the invention, an isolated peptide tetramer non-proteogenic amino acid modification may comprise (i) including at least one additional non-proteogenic amino acid at any position of and/or (ii) substituting at least one amino acid residue with a non-proteogenic amino acid in; at least one peptide monomer.

In particular, an additional non-proteogenic amino acid may be included at any position of at least one peptide monomer.

The isolated peptide tetramer or isolated peptide tetramer derivative according to any aspect of the invention may be for use as a medicament, pharmaceutical composition and/or antimicrobial composition and/or in therapy. The antimicrobial composition may be an antibacterial, antifungal or anti-protozoan composition. In particular, the antimicrobial composition comprises an antifungal composition.

The invention includes the use of an isolated peptide tetramer or isolated peptide tetramer derivative according to any aspect of the invention in the preparation of a medicament for preventing and/or treating at least one microbial infection. The microbial infection may be selected from the group consisting of bacterial infection, fungal infection and protozoan infection. In particular, the microbial infection comprises a fungal infection.

The invention also includes a method of inhibiting and/or reducing the growth of at least one microorganism comprising contacting the microorganism with at least one isolated peptide tetramer or isolated peptide tetramer derivative according to any aspect of the invention. This method may be an *in vitro* method. The microorganism may be selected from the group consisting of bacteria, fungi and protozoa. In particular, the microorganism comprises a fungi.

The invention also includes a method of preventing and/or treating at least one microbial infection comprising administering at least one isolated peptide tetramer or isolated peptide tetramer derivative according to any aspect of the invention.

The invention further includes a contact lens and/or eye drop solution, a pharmaceutical and/or antimicrobial composition, a composition for coating a device and/or a kit comprising the isolated peptide tetramer or isolated peptide tetramer derivative according to any aspect of the invention.

Methods of preparing the isolated peptide tetramer or isolated peptide tetramer derivative are included as part of the invention.

In one example, the method of preparing a peptide tetramer comprising four identical peptide monomers or isolated peptide tetramer derivative comprising four identical peptide monomers, comprises the steps of:

(i) providing at least one solid phase;
(ii) coupling a first protected K residue to the solid phase;
(iii) removing a protective group from one amine group in the first K residue;
(iv) linking a second protected K residue to the first K residue;
(v) removing a protective group from one amine group in the second K residue;
(vi) linking two protected K residues to the second K residue;
(vii) removing the protected groups from the two linked K residues;
(viiii) providing additional chain extension by linking protected amino acid residues; according to the sequence of the respective peptide monomer from the C-terminus to the N-terminus, wherein after each linking, the protecting groups are moved for the next linking;
(ix) terminating the linking of amino acid residues depending on the number of residues to be added; and
(x) optionally, releasing the multimer from the solid phase.

In another example, the method of preparing a peptide tetramer comprising four identical peptide monomers or isolated peptide tetramer derivative comprising four identical peptide monomers, comprises the steps of:

(i) providing at least one solid phase;
(ii) coupling a first protected K residue to the solid phase;
(iii) removing a protective group from one amine group in the first K residue;
(iv) linking a second protected K residue to the first K residue;
(v) removing a protective group from one amine group in the second K residue;
(vi) linking two protected K residues to the second K residue;
(vii) removing the protected groups from the two linked K residues;
(viii) linking one peptide monomer with a protected terminal amine group to each amine group from the two K residues; and
(iv) optionally, releasing the multimer from the solid phase.

According to another aspect, the method of preparing a peptide tetramer comprising four identical peptide monomers or isolated peptide tetramer derivative comprising four identical peptide monomers comprises the steps of:

(i) providing a least one solid phase;
(ii) coupling a first K residue to the solid phase;
(iii) linking two protected K residues to the coupled first K residue;
(iv) removing the protective groups from the linked K residues;
(v) providing additional chain extension by linking protected amino acid residues, according to the sequence of the respective peptide monomer from the C-terminus to the N-terminus, wherein after each linking, the protecting groups are removed for the next linking;
(vi) terminating the linking of amino acid residues depending on the number of residues to be added; and
(vii) optionally, releasing the multimer from the solid phase.

Further, the method of preparing a peptide tetramer comprising four identical peptide monomers or isolated peptide tetramer derivative comprising four identical peptide monomers comprises the steps of:

(i) providing a least one solid phase;
(ii) coupling a first K residue to the solid phase;
(iii) linking two protected K residues to the coupled first K residue;
(iv) removing the protective groups from the linked K residues;
(v) linking one peptide monomer with a protected terminal amine group to each amine group from the two K residues; and
(vi) optionally, releasing the multimer from the solid phase.

Having now generally described the invention, the same will be more readily understood through reference to the following examples which are provided by way of illustration, and are not intended to be limiting of the present invention.

## EXAMPLES

Standard molecular biology techniques known in the art and not specifically described were generally followed as described in Green, Molecular Cloning: A Laboratory Manual, Cold Springs Harbor Laboratory, New York (2012).

### Example 1: B4010 tetrameric peptide and scrambled B4010

The peptide monomer RGRKWRRKK (SEQ ID NO: 27), linear retrodimer RGRKVVRRKKKRRWKRGR (SEQ ID NO: 28), the peptide dimer B2088 $(RGRKVVRR)_2KK$, the peptide tetramer B4010 $[(RGRKVVRR)_2K]_2KK$ and scrambled B4010 $[(VRGRVRKR)_2K]_2KK$ are illustrated in Figure 1. The structure of nantamycin is also shown in Figure 1.

*Modified peptides*

In this procedure, every amino acid in the B4010 sequence (RGRKWRR) is replaced with alanine residue and the antimicrobial and haemolytic activities will be evaluated by high throughput screening (HTS) methods. In the second method, every amino acid residue in B4010 sequence will be successively deleted and their activity-toxicity profile will be evaluated as before. After evaluating the MIC of all the peptides against at least three strains, the best peptides will be chosen and assessed for their activity in 50% rabbit tear fluid, 25% serum and in trypsin (enzyme: peptide ~ 1:100). Table 1 shows the modified peptides and their properties.

Table 1 Modified peptides

| **Peptide** | **Peptide sequence** | **Overall Net Charge** |
|---|---|---|
| B4010 | $[(RGRKVVRR)_2K]_2KK$<br>$[(SEQ\ ID\ NO:\ 1)_2K]_2KK$ | +24 |
| B4010-R1A | $[($**A**$GRKVVRR)_2K]_2KK$<br>$[(SEQ\ ID\ NO:\ 2)_2K]_2KK$ | +20 |
| B4010-G2A | $[(R$**A**$RKVVRR)_2K]_2KK$<br>$[(SEQ\ ID\ NO:\ 3)_2K]_2KK$ | +24 |

(continued)

| Peptide | Peptide sequence | Overall Net Charge |
|---|---|---|
| B4010-R3A | $[(RG\mathbf{A}KVVRR)_2K]_2KK$<br>$[(SEQ\ ID\ NO{:}\ 4)_2K]_2KK$ | +20 |
| B4010-K4A | $[(RGR\mathbf{A}VVRR)_2K]_2KK$<br>$[(SEQ\ ID\ NO{:}\ 5)_2K]_2KK$ | +20 |
| B4010-V5A | $[(RGRK\mathbf{A}VRR)_2K]_2KK$<br>$[(SEQ\ ID\ NO{:}\ 6)_2K]_2KK$ | +24 |
| B4010-V6A | $[(RGRKV\mathbf{A}RR)_2K]_2KK$<br>$[(SEQ\ ID\ NO{:}\ 7)_2K]_2KK$ | +24 |
| B4010-R7A | $[(RGRKVV\mathbf{A}R)_2K]_2KK$<br>$[(SEQ\ ID\ NO{:}\ 8)_2K]_2KK$ | +20 |
| B4010-R8A | $[(RGRKVVR\mathbf{A})_2K]_2KK$<br>$[(SEQ\ ID\ NO{:}\ 9)_2K]_2KK$ | +20 |
| B4010-R3AK4A | $[(RG\mathbf{AA}VVRR)_2K]_2KK$<br>$[(SEQ\ ID\ NO{:}\ 10)_2K]_2KK$ | 16 |
| B4010-R7AR8A | $[(RGRKVV\mathbf{AA})_2K]_2KK$<br>$[(SEQ\ ID\ NO{:}\ 11)_2K]_2KK$ | 16 |
| B4010-R3AV5A | $[(RG\mathbf{A}K\mathbf{A}VRR)_2K]_2KK$<br>$[(SEQ\ ID\ NO{:}\ 12)_2K]_2KK$ | 20 |
| B4010-V5AV6A | $[(RGRK\mathbf{AA}RR)_2K]_2KK$<br>$[(SEQ\ ID\ NO{:}\ 13)_2K]_2KK$ | 24 |
| B4010- R3AK4AV5A | $[(RG\mathbf{AA}A\mathbf{V}RR)_2K]_2KK$<br>$[(SEQ\ ID\ NO{:}\ 14)_2K]_2KK$ | 16 |
| B4010-R3AV5AV6A | $[(RG\mathbf{A}K\mathbf{AA}RR)_2K]_2KK$<br>$[(SEQ\ ID\ NO{:}\ 15)_2K]_2KK$ | 20 |
| B401 0-K4AV5AV6A | $[(RGRAAARR)_2K]_2KK$<br>$[(SEQ\ ID\ NO{:}\ 16)_2K]_2KK$ | 20 |
| B4010-R3K4V5V6 | $[(RG\mathbf{AAAA}RR)_2K]_2KK$<br>$[(SEQ\ ID\ NO{:}\ 17)_2K]_2KK$ | 16 |
| B4010-V5V6R7R8 | $[(RGRK\mathbf{AAAA})_2K]_2KK$<br>$[(SEQ\ ID\ NO{:}\ 18)_2K]_2KK$ | 16 |
| Sc_B4010 | $[(VRGRVRKR)_2K]_2KK$<br>$[(SEQ\ ID\ NO{:}\ 19)_2K]_2KK$ | 24 |
| B4010-Δ1 | $[(GRKVVRR)_2K]_2KK$<br>$[(SEQ\ ID\ NO{:}\ 20)_2K]_2KK$ | +20 |
| B4010-Δ1,2 | $[(RKVVRR)_2K]_2KK$<br>$[(SEQ\ ID\ NO{:}\ 21)_2K]_2KK$ | +20 |
| B4010-Δ1-3 | $[(KVVRR)_2K]_2KK$<br>$[(SEQ\ ID\ NO{:}\ 22)_2K]_2KK$ | +16 |
| B4010-Δ1-4 | $[(VVRR)_2K]_2KK$ | +12 |
| | $[(SEQ\ ID\ NO{:}\ 23)_2K]_2KK$ | |
| B4010-V1-5 | $[(VRR)_2K]_2KK$<br>$[(SEQ\ ID\ NO{:}\ 24)_2K]_2KK$ | +12 |

(continued)

| Peptide | Peptide sequence | Overall Net Charge |
|---|---|---|
| B4010-Δ1-6 | $[(RR)_2K]_2KK$ <br> $[(SEQ\ ID\ NO: 25)_2K]_2KK$ | +12 |
| B4010-Δ1-7 | $[(R)_2K]_2KK$ <br> $[(SEQ\ ID\ NO: 26)_2K]_2KK$ | +8 |

After identifying the peptides that display excellent activity in complex biological fluid, the following modifications by non-proteinogenic amino acid residues exemplified in Table 2 will be chosen.

Table 2 Non-proteinogenic amino acid residues for modification

| Name | Altered Properties | Structure |
|---|---|---|
| | **Charge** | |
| Ornithine (Orn) | Charge and Side chain depth | One $-CH_2-$ less than lysine |
| Diamino butyric acid (Dab) | | Two $-CH_2-$ less than lysine |
| Diamino propionic acid (Dap) | | Three $-CH_2-$ less than lysine |
| Homo arginine (Harg) | | One $-CH_2-$ more than arginine |
| α-amino guanidino propionic acid (Agp) | | Two $-CH_2-$ less than arginine |
| | **Hyrophobicity** | |
| Norleucine (Nle) | | Linear n-butyl side chain compared isobutyl side chain in leucine |
| Norvaline (Nva) | | Linear n-propyl side chain compared isopropyl side chain in leucine |
| Aminobutyric acid (Abu) | | One $-CH_2-$ more than alanine |
| Dipropyl glycine (Dpg) | | Two propyl group attached to the □-Carbon atom of the amino acid |
| Diethyl glycine (Deg) | | Two ethyl group attached to the □-Carbon atom of the amino acid |
| Biphenyl alanine (Bip) | | An extra aromatic ring attached to the $4^{th}$ position of phenyl alanine |
| β-(benzothaien-3-yl)alanine (Bal) | | A sulfur group instead of nitrogen in tryptophan side chain. |
| 1-(Naphth-1-yl)alanine (1-Nal) | | An α-naphthyl group is substituted in the side chain |
| 2-(Naphth-1-yl)alanine (2-Nal) | | An α-naphthyl group is substituted in the side chain |
| β -diphenyl alanine (Dip) | | Two phenyl groups are attached to β-carbon of alanine |
| β-(anthracen-9-yl)alanine (Ath) | | $9^{th}$ position of anthracene is attached to β-carbon of alanine |
| β -(2,5,7-tri-tert-butyl-indol-3-yl) alanine (Tbt) | | Three t-butyl groups are attached at the 2,5, and $7^{th}$ position of tryptophan |

(continued)

| | Conformational stability | |
|---|---|---|
| α-aminobutyric acid (Aib) | | Two methyl group attached to the α-Carbon atom of the amino acid |
| 1-amino cyclopentane carboxylic acid (Acp) | | Structural analogue of proline. The cyclopentyl group is not a part of the backbone |
| Hydroxyl prole (Hyp) | | An -OH group is attached to the proline ring |
| | Peptoids | |
| N-(4-aminobutyl)glycine | | Lysine analogue |
| N-methylimidazole glycine | | Histidine analogue |

Example 2: Chemicals and Peptides

Chemicals and Peptides: Sabouraud's Dextrose Agar was purchased from Acumedia (Michigan, MI, USA). Peptides were purchased from EZBiolabs Inc., (Carmel, IN, USA). Lipids such as L-α-phophatidylcholine (PC), L-α-phostidylethanolamine (PE), L-α-phosphatidylserine (PS) and L-α-phosphatidylinositol (PI) were bought from Avanti Polar Lipids Inc. (AL, USA). Ergosterol, sodium azide ($NaN_3$), Carbonyl cyanide m-chloro phenylhydrazone (CCCP), 4-aminopyridine (4-AP), 5-nitro-2-(3-phenylpropylamino) benzoic acid (NPPB), Gadolinium III Chloride, tetra ethyl ammonium chloride (TEA) and 3',3'-dipropylthiadicarbocyanine (diS-C3-5) dye were purchased from Sigma-Aldrich Corp. (MO, USA). ATP bioluminescence kit was obtained from Molecular Probes Inc (OR, USA). Amphotericin B and Natamycin were obtained in powder form from Sigma-Aldrich (S) Pte Ltd (Singapore).

Example 3: Circular Dichroism (CD) Spectropolarimetry

Far UV-CD spectra of the peptides (0.6 mg/ml) were recorded on a JASCO J810 spectropolarimeter (JASCO, Tokyo, Japan) in 10 mM PBS (pH 7.0) using a 0.1 cm path length quartz cuvette at 25 °C. Spectra were recorded from 260 nm to 190 nm in 0.1 nm steps at a scan rate of 50 nm/min. The final spectrum is the average of 4 scans. The CD data is expressed as mean residual ellipticity ((θ)mrw, deg $cm^2$ $dmol^{-1}$).

Example 4: Antifungal activity of multivalent peptides:

*Determination of minimum inhibitory concentration (MIC)*

The yeast strains were cultivated and suspended in Sabouraud's Dextrose (SD) broth diluted at one sixth at a starting $OD_{600}$ = ~0.08 in a flat-bottomed microtitre plate. A serial dilution of peptide in the same broth was mixed with the inoculum to give a final peptide concentration of 0.4 - 22 μM. The antifungal activity was assessed by monitoring the $OD_{600}$ in cycles of 30 minutes and an orbital shaking at 100 rpm using an Infinite M200 microplate reader (Tecan Group Ltd., Switzerland) for 24/48 h at 37°C. Cultures without peptides were used as positive controls and broth alone or with 22 μM peptide served as negative controls. The minimum concentration required for complete inhibition was assessed by both visible observations as well as by measuring the OD600 and taken as the MIC. Each experiment was repeated in triplicates.

*Results:* The peptide monomer, RGRKVVRRKK (SEQ ID NO: 27) and the linear retrodimer RGRKWRRKKKRRVVKRGR (SEQ ID NO: 28) displayed weak antifungal activity (Table 3) against both *C. albicans* and *Fusarium* strains. However, upon linking two copies of the sequence through a branched lysine (B2088, Figure 1), substantial (~14×) decrease in MIC values were observed as compared to the monomer (Table 3). Further assembling the two copies of branched dimer through branched lysines produced a tetrabranched peptide (B4010) which decreased the MIC by an additional 4-10 fold compared to the co-valent dimer (Table 3). The MIC values of B4010 (0.34 μM) for two *C. albicans* clinical isolates were also lower when compared to the MIC values for amphotericin B (1.4 μM) and natamycin (15 μM), the only US FDA approved antifungal for ophthalmic applications. Interestingly, scrambling the sequence of B4010 (Sc_B4010) led to 2-4-fold increase in MIC values, yet retained significant potency. CD spectra displayed strong negative minima around 200 nm for both the tetrabranched peptides, suggesting an unordered conformation for both the peptides (Figure 8).

The peptide B4010 displayed a broad spectrum antifungal activity against ATCC reference isolates as well as

clinical isolates. (Table 3). The activity was 2-4 times superior to natamycin, the only US FDA approved ophthalmic drug. Table 4 shows the MIC and cytotoxicity of other designed peptides.

Table 3 Minimum Inhibitory Concentration (MIC) values of peptides and natamycin against various fungi

| **Strains of Yeast/Fungi** | **MIC in μM of** | | | | | |
|---|---|---|---|---|---|---|
| | **Monomer[1]** | **Linear Retrodimer** | **B2088** | **B4010** | **Sc_B4010** | **Natamycin** |
| *C. albicans ATCC10231* | 78 | 22 | 5.5 | 1.4 | 5.6 | 15 |
| *C. albicans ATCC24433* | n.d | 5.5 | 2.7 | 1.4 | 5.6 | 15 |
| *C. albicans ATCC2091* | n.d | 11 | 1.4 | 0.7 | 2.7 | 7.5 |
| *C. albicans DF2672R* | 78 | 2.7 | 0.8 | 0.34 | 0.8 | 15 |
| *C. albicans DF1976R* | n.d. | 2.7 | 0.8 | 0.34 | 0.8 | 15 |
| *F. solani ATCC36031* | >78 | n.d. | n.d. | 1.4 | n.d. | 4.7 |
| *F. solani DM3782* | n.d. | n.d. | n.d. | 1.4 | n.d. | 2.4 |
| *F. solani DF1500* | n.d. | n.d. | n.d. | 1.4 | n.d. | 2.4 |
| n.d. - not determined | | | | | | |

Table 4 MIC and cytotoxicity of various designed tetrameric peptides

| **Peptide** | **Minimum Inhibitory Concentration (μM) against** | | | | | **$EC_{50}$, μM[a]** |
|---|---|---|---|---|---|---|
| | ***C. albicans* ATCC 10231** | ***C. albicans* ATCC 24433** | ***C. albicans* ATCC 2091** | ***C. albicans* DF2672R** | ***C. albicans* DF1976R** | |
| [(SEQ ID NO: 1)$_2$K]$_2$KK | 1.4 | 1.4 | 0.7 | 0.37 | 0.37 | 220 |
| [(SEQ ID NO: 2)$_2$K]$_2$KK | 5.9 | 5.9 | 1.5 | 1.5 | 0.75 | >237 (67.6)b |
| [(SEQ ID NO: 3)$_2$K]$_2$KK | 1.3 | 1.4 | 0.7 | 0.4 | 0.16 | 110 |
| [(SEQ ID NO: 4)$_2$K]$_2$KK | 1.5 | 1.5 | 1.5 | 0.4 | 0.4 | >237 (80.9%) b |
| [(SEQ ID NO: 5)$_2$K]$_2$KK | 1.4 | 2.8 | 1.4 | 1.4 | 0.34 | >230.7 (59.9%) |
| [(SEQ ID NO: 6)$_2$K]$_2$KK | 1.4 | 1.4 | 0.7 | 0.4 | 0.17 | 190.4 |
| [(SEQ ID NO: 7)$_2$K]$_2$KK | 1.4 | 1.4 | 0.7 | 0.7 | 0.4 | >225 (53.4%) |
| SEQ ID NO: 8)$_2$K]$_2$KK | 0.74 | 0.7 | 0.7 | 0.7 | 0.4 | >237 (73.9%) |
| [(SEQ ID NO: 9)$_2$K]$_2$KK | 1.4 | 1.4 | 0.7 | 1.4 | 0.7 | >237 (68.7%) |
| [(SEQ ID NO: 10)$_2$K]$_2$KK | 3.12 | 1.6 | 0.8 | 0.8 | 0.8 | 250 |

(continued)

| Peptide | Minimum Inhibitory Concentration (μM) against | | | | | $EC_{50}$, μM[a] |
|---|---|---|---|---|---|---|
| | *C. albicans* ATCC 10231 | *C. albicans* ATCC 24433 | *C. albicans* ATCC 2091 | *C. albicans* DF2672R | *C. albicans* DF1976R | |
| [(SEQ ID NO: $11)_2K]_2KK$ | 0.8 | 1.6 | 0.8 | 0.8 | 0.4 | >257 (73%) |
| [(SEQ ID NO: $12)_2K]_2KK$ | 6.1 | 3.0 | 0.8 | 0.38 | 0.38 | >243 (81.5%) |
| [(SEQ ID NO: $13)_2K]_2KK$ | 2.9 | 1.8 | 0.72 | 0.18 | 0.18 | 46 |
| [(SEQ ID NO: $14)_2K]_2KK$ | 1.6 | 0.4 | 0.4 | 0.2 | 0.4 | >257 (76.5) |
| [(SEQ ID NO: $15)_2K]_2KK$ | 1.6 | 0.4 | 0.4 | 0.2 | 0.2 | >250 (56.4) |
| [(SEQ ID NO: $16)_2K]_2KK$ | 0.8 | 0.38 | 0.8 | 0.38 | 0.38 | 169 |
| [(SEQ ID NO: $17)_2K]_2KK$ | 0.85 | 0.2 | 0.41 | 0.21 | 0.21 | >265 (61.2) |
| [(SEQ ID NO: $18)_2K]_2KK$ | 0.85 | 0.85 | 0.85 | 0.4 | 0.4 | >273 (82%) |

Example 5: Time-kill kinetics assay

The time-kill kinetics was measured for two strains of *C. albicans* (ATCC 10231 and DF2672R). The cultures were grown overnight in SD broth and the cell concentration was adjusted to $10^5$-$10^6$ CFU/ml with phosphate buffer. The peptide or antifungals were added to the individual cultures. Appropriate final concentrations of B4010 (1.4 - 11 μM for ATCC and 0.37-3.7 μM for CA 2672R) or amphotericin B (0.55 μM - 11 μM for CA 2672R strains) and natamycin (4.7 μM - 75 μM for ATCC and 30 μM for CA 2672R strains) were adjusted for the inocula. The test solution was incubated at 37 °C with constant shaking. 100 μL of the suspension is withdrawn at predetermined time points, serially diluted ($10^2$ or $10^3$ fold) and poured into the SDA plate. The plate was incubated for 48 h at 37 °C for colony counting. The data were expressed in terms of % cell viability relative to the positive control.

Figure 2A and Figure 2B show the results of the concentration-dependent time-kill experiments conducted after exposing *C. albicans* to B4010 and polyene antifungals. For the ATCC strains, B4010 induced ~91% killing in 1 h at 1× MIC whereas ~97% killing was observed as the concentration was increased to 2× MIC. Increasing the concentration of B4010 to 4× and 8× MIC values led to 4 log reduction (99.99%) in viable cells within 20 minutes and 10 minutes, respectively. In contrast, natamycin, even at 16× MIC, required 24 h to achieve similar endpoints. The kill-kinetics of B4010, natamycin and amphotericin B against the clinical isolates *C. albicans DF2672R* strains were also compared. At 2× MIC, B4010 elicited complete killing in 1 h whereas for amphotericin B maximal effect was achieved in less than 8 h at 16× MIC. However, no fungicidal action was observed for natamycin at 2× MIC even after 24 h exposure.

Example 6: Effect of monovalent and divalent cations on the antifungal activity of B4010

For studying the effects of metal ions, SD broth was adjusted with appropriate concentrations of salts to yield a final concentration of 100 and 135 mM for NaCl and KCl and 0.5-2 mM for $CaCl_2$ and $MgCl_2$. MIC determinations were carried out as described above. *C. albicans* ATCC10231 cells and salts in broth were taken as a positive control. Salts alone (NaCl, KCl, and $CaCl_2$ and $MgCl_2$) in broth or with 22 μM peptide served as a negative control.

To study the effects of metal ions on viability, the broth solution containing *C. albicans* ($OD_{600}$ = 0.4-0.6) was adjusted to appropriate final concentrations of metal ions, incubated with 5.5 μM of B4010 for 6 h and the cell viability was determined as before after 48 h.

Results: The effects of physiological concentrations of metal ions (NaCl, $CaCl_2$ and $MgCl_2$) on the antifungal activity of B4010 were examined. The growth of *C. albicans* ATCC 10231 was monitored at various concentrations of

the peptide and in the presence of physiologically relevant (close to the concentration present in tear fluid) concentrations of monovalent and divalent cations (Figure 3A and 3B). Addition of 100 and 135 mM NaCl appeared to ameliorate the antifungal activity of B4010 as no visible growth was observed at 3.125 μg/ml (Figures 3A). The presence of 135 mM NaCl ameliorated the antifungal activity, decreasing the MIC by 2-fold whereas physiological concentrations of divalent cations resulted in only a 2-fold increase in MIC values (Figure 3A). A high concentration of $Ca^{2+}$ and $Mg^{2+}$ ions lead to two fold increase in the MIC values. However, potassium had dramatic effect on the antifungal activity of B4010 as an 8 fold increase in MIC values was observed when 25 mM KCl was present (Figure 3B). Addition of 100 mM KCl resulted in an increase in MIC by a factor of >16 compared to the medium not supplemented with KCl. The cation sensitivity on the candidacidal activity of B4010 was also determined. Addition of B4010 (at 5.5 μM) resulted in a complete loss of viable cells in the presence of monovalent and divalent cations (Figure 3B). However, in the presence of a high concentration of KCl, a slight decrease in the candidacidal activity was observed.

Example 7: Effect of trypsin, serum and tear fluid on antifungal activity of B4010.

*Antifungal activity of B4010 in presence of trypsin and 50% tear fluid*

For all the experiments, unless otherwise stated, ATCC 10231 strain was used. B4010 (1 mg/ml) was incubated with trypsin (enzyme:peptide ratio 1:100) at 37 °C. 20 μL aliquot of this mixture was withdrawn at various time intervals (0.5, 1, 2, 4 and 6 h) and mixed with 1 μL of trypsin inhibitor. The antifungal activity of the peptide was determined by adding this mixture to 180 μl of inoculum and monitoring the growth at $OD_{600}$ for 24 h. Similar experiments performed in the presence of trypsin/trypsin inhibitor (without B4010) were used as a positive control. For assessing the antifungal activity in tear fluid (TF), the peptide was dissolved in freshly collected rabbit TF and incubated at 37 °C for 6 h. After incubation, an equal volume of an overnight culture of *C. albicans* (~$10^6$ CFU/ml) was mixed with peptides in tear fluid and incubated at 37 °C for 24 h. The final concentrations of the peptide were at 4.4, 8.8 and 22 μM. A 100 μL aliquot of serial dilutions ($10^2$ or $10^3$ times) of this mixture were inoculated on a SD agar plate and then incubated for 48 h at 37 °C. Culture alone and culture with 50% tear fluid served as the positive control. The data was expressed in % killing with respect to culture without tear fluid. About 6-10% killing was observed in the presence of 50% tear fluid.

*Determination of MIC in 5% human serum*

The effect of 5% human serum on the activities of B4010 against two *C. albicans* clinical isolates was determined. The human male serum was centrifuged at 13,000 rpm for 10 mins in order to remove the lipids and the supernatant was collected. The MIC values against clinical isolates of *C. albicans* 2672R and *C. albicans* 1976R were determined both in standard medium (SD broth) as well as in standard medium containing 5% serum supernatant as before.

Antifungal properties of the peptide were examined in three complex biological environments.

*Trypsin*

The peptide B4010 was incubated with trypsin (trypsin:peptide ratio = 1:100) at 37 °C. An aliquot of this mixture was withdrawn at various time intervals, added to the *C. albicans* inoculum and the growth was monitored (Figure 3C). No loss of antifungal activity was found up to 6 h incubation with trypsin, suggesting improved proteolytic resistance of tetrabranched peptides.

*Tear fluid and human serum*

The antifungal efficacy of B4010 was also monitored in the presence of complex biological fluids such as tear fluid and human serum.

*Tear fluid*

The antifungal activity of B4010 was assessed in 50% tear fluid. In the absence of tear fluid, about 98 ± 2% loss of viable cells were observed at a peptide concentration of 5.5 μM. However, the candidacidal activity was moderately suppressed by the presence of 50% tear fluid at lower (4.4 and 8.8 μM of B4010) concentration (Figure 3D). At 22 μM, however, no loss of activity was observed suggesting a higher concentration of peptide was needed to achieve more than a 3 log reduction of *C. albicans* in the presence of tear fluid.

*Serum*

The effect of serum on antifungal efficacy of B4010 was assessed by measuring the change in the MIC of B4010 in the presence of 5% human serum against two clinical isolates of *C. albicans.* For both the strains the MIC values were shifted from 0.34 μM without serum to 5.5 μM in the presence of serum, suggesting 16-fold increase in the MIC in 5% sera.

Example 8: Effect of B4010 on haemolysis, cytotoxicity, corneal reepithelialization rate and *in vivo* toxicity

*Haemolytic assay*

Haemolytic activity of peptides and antifungal drugs were determined against rabbit red blood cells (Oren *et al.,* 1997). Briefly, serial dilution of peptides/antifungals in PBS was mixed with rRBC (final concentration 4% v/v), incubated at 37 °C for 1 h and centrifuged at 3000 rpm for 10 minutes. The release of hemoglobin in the supernatant was monitored by measuring the hemoglobin absorbance at 576 nm. The readings from cell suspension in PBS (without any additives) or 1% Triton-X100 were used as 0% or 100% haemolysis.

Results of haemolytic assay: The ability of B4010 in disrupting the membrane integrity of mammalian cells was evaluated by haemolytic assay using rabbit red blood cells. The peptide had no significant haemolytic activity (<1 % haemolysis) against rabbit erythrocytes even at 440 μM (Figure 4A). On the other hand amphotericin B and natamycin displayed significant haemolytic activity at low concentration and the $HC_{50}$ values were 59.4 ± 7.8 μM and 139.6 ± 0.18 μM, respectively.

Table 5 summarises of the haemolysis assay of various peptide tetramers

Table 5 Haemolysis Assay

| Peptide, 200 μg/ml | % Haemolysis |
|---|---|
| B4010 | 1% |
| B4010-R1A | No haemolysis |
| B4010-G2A | No haemolysis |
| B4010-R3A | No haemolysis |
| B4010-K4A | No haemolysis |
| B4010-V5A | No haemolysis |
| B4010-V6A | No haemolysis |
| B4010-R7A | No haemolysis |
| B4010-R8A | No haemolysis |
| 2% triton-X100 | 100% |

*Cytotoxicity on human conjunctival epithelial cells*

IOBA-NHC cells were cultured under standard conditions (humidified atmosphere of 5% CO2 at 37°C) in DMEM/F12 supplemented with 1 μg/ml bovine pancreas insulin, 2 ng/ml mouse epidermal growth factor, 0.1 μg/ml cholera toxin, 5 μg/ml hydrocortisone, 10% fetal bovine serum (FBS), 50 Ul/ml penicillin, and 50 Ul/ml streptomycin. Cells from passages 50-80 were used in all experiments. Every day, normal culture development was observed by phase-contrast microscopy. Cells were removed by gentle trypsin incubation at confluence and counted. They were seeded into 96 well culture plates (Corning, Schiphol-Rijk, The Netherlands) for microtitration analysis of flow cytotoxicity assay (~10,000 cells/well). Cultures were kept at 37°C for 24 h. Subconfluent cells (culture surface covering nearly 70%) were then exposed to various peptide concentrations (0.22 - 225 μM). Cytotoxicity was measured periodically using MultiTox-Fluor Multiplex assay kit (Promega, WI, USA) by measuring the AFC fluorescence (λex = 485 and λem = 520 nm). Cell viability was reported after 24 h incubation of the cells with the peptides since there was no detectable toxicity even after 8 h of incubation and the $EC_{50}$ (effective concentration of peptide that reduces the viable cells by 50%) was determined.

*In vivo* cytotoxicity

Acute toxicity of B4010 was assessed with C57BL6 (6-8 weeks old) wild type mice. Two healthy wild type mice were chosen for each route of administration. B4010 was delivered through intra-peritoneal (200 mg/kg) or (100 mg/kg) intravenous routes. Two animals were used for each administration and monitored through 24/48 h to determine mortality, or signs of toxicity.

*Results of cytotoxicity assay:* Exposure of B4010 caused 50% loss of cell viability at 220 μM (Figure 4B). The $EC_{50}$ value of B4010 was comparable to natamycin (211.7 ± 20.5 μM) and better than amphotericin B (134 ± 16 μM) (Figure 4B).

*Rabbit model of corneal wound healing*

It was further examined whether topical application of B4010 would influence the corneal wound healing *in vivo.* All animal experiments were conducted in compliance with the ARVO statement for the Use of Animals in Ophthalmic and Vision Research, the guide for the Care and Use of laboratory animals (National Research Council) and under the supervision of Singhealth Experimental Medical Centre (SEMC). Four New Zealand white rabbits were divided into two groups, two each for control (saline) and two each for the study group treated with B4010. Rabbits were tranquilized by intra muscular injection of 1 ml of ketamine (100 mg/ml) and 0.5ml of xylazil (20 mg/ml). Corneas were anesthetized by topical administration of 1% xylocaine. A 5-mm trephine was used to outline the wound margin and mechanical removal of epithelial cells was carried out by sterile mini blade (BD-Beaver) leaving the basal lamina intact as previously described (Crosson *et al.,* 1986). The animals were treated by topical administration of 22 μM of B4010, 3 times per day. Cornea wound was visualized by staining with fluorescein sodium, which is used in the ophthalmology clinic as a non-toxic dye to disclose wounds of the cornea, and using a cobalt-blue filter with slit lamp biomicroscopy. Measurements of the residual wound area were performed during the reepithelialization process by Image-J 1.440.

Results: The epithelial wound healing rate was not altered by topical application of B4010 (22 μM) 3 times a day compared to the control groups (Figure 4C) This is an important result as corneal epithelial wound healing re-establishes a critical component of innate immunity for the eye. Preliminary *in vivo* toxicity tests on mice indicated no sign of mortality, morbidity or toxicity when injected by IP (200 mg/kg) or IV (100 mg/kg) routes.

Example 9: Antifungal activity of B4010 against *ergΔ strains*

*Determination of MIC against wild type (WT) and mutant S. cerevisiae strains*

The antifungal activity of B4010 was also tested against *S. cerevisiae* erg*Δ* mutants carrying an altered sterols structure and composition.

A few identical colonies from WT and mutant strains were immediately inoculated and grown overnight in SD broth containing 0.1 mg/ml ampicilin at 30 °C. Cells were harvested by centrifugation, washed with sterile water and frozen. Cells were plated again on SD agar plates and incubated at 37 °C for 48 h. Two or three identical colonies of the mutant strains were collected and inoculated in SD broth. Peptides were dissolved in sterile water and mixed with WT and mutant strains and incubated at 37 °C for 48 or 72 h in a test tube. The lowest concentration of the peptide that inhibited the growth of the yeast strain was determined by visual inspection.

Results: The emergence of resistant pathogens poses a greater threat to the management of fungal infections. To determine if the sterol alterations have an affect on the antifungal activity of B4010, experiments were performed using *S. cerevisiae* strains that carry specific mutations in the ergosterol (*ergΔ*) pathways. Table 6 summarizes the MIC values of B4010 against wild type and various *ergΔ* mutants which carry altered sterol structure and compositions. Interestingly, all the *ergΔ* mutants displayed hyper susceptibility to B4010 i.e. 2-4 fold decrease in MIC values compared to the wild type strains.

*ErgΔ* mutants are hypersensitive to B4010. As a result of the mutation, the strains accumulate sterols with altered structure in B or C ring of ergosterol and the side chains. For simplicity, the major sterol is given in Table 5 for the strains. Overall, the mutant strains displayed 2-4 fold decrease in MIC values compared to the wild type strain. The mutant strains *erg2Δ, erg3Δ, erg3Δerg6Δ* and *erg4Δerg5Δ* displayed increased sensitivity to B4010 (4 folds decrease in MIC compared to the wild type). The mutant strains *erg2Δerg6Δ* and *erg6Δ* displayed 2 fold increased sensitivity to B4010.

Table 6 MIC values of B4010 against S. *cerevisiae* strains containing various sterol structure and composition

| Yeast strain | Relevant genotype | Sterol composition (%) | MIC (μM) |
|---|---|---|---|
| RH448 | Wildtype | Ergosterol (77%) | 5.5 |

(continued)

| Yeast strain | Relevant genotype | Sterol composition (%) | MIC (μM) |
|---|---|---|---|
| RH3616 | *erg2Δerg6Δ* | Zymosterol (86%) | 2.8 |
| RH4213 | *erg3Δ* | Ergosta-7,22-dienol (46%) | 1.4 |
| RH5684 | *erg6A* | Zymosterol (39.4%) Cholesta-5,7,24-trienol (32.3%) | 2.8 |
| RH5930 | *erg3Δerg6Δ* | Zymosterol (40.9%) Cholesta-5,7,24-trienol (39.5%) | 1.4 |
| RH5873 | *erg4Δerg5Δ* | Ergosta-5,7,24-trienol (72.2%) | 1.4 |
| RH5812 | *erg2Δ* | Ergosta-8,24(28)-dienol (23.8%) | 1.4 |
| RH6827 | | Campasterol | 2.8 |
| RH6829 | | Cholesterol | 2.8 |
| RH6826 | | 7-deoxycholesterol | 2.8 |

Example 10: Interaction with outer cell wall components

*SDS-PAGE pull down assays*

In another study to probe the mechanism of action, the affinity of B4010 for insoluble polysaccharides such as chitin and β-D-glucan, the major component of fungal cell walls was examined. B4010 (5.5 - 22 μM) was incubated with chitin (from shrimp shells) or β-D-glucan for 2 h at 37 °C. The mixture was centrifuged at 10,000 g and the supernatant was analyzed by SDS-PAGE (4-20%). A control experiment without chitin/ β -D-glucan was also performed to quantify peptide binding to the carbohydrate polymers.

The peptide showed no affinity for cell wall polysaccharides as no coprecipitation was observed with either chitin or β-D-glucan (Figure 5A).

Example 11: Membrane disrupting activity of B4010

Two complementary fluorometry assays were performed to determine if B4010 has a membrane targeting action.

*SYTOX Green (SG) uptake assays*

To check if the peptide permeabilized the cytoplasmic membrane, the uptake of SG was assayed. This is a membrane impermeable dye which does not fluoresce when incubated with cells which have intact cell membrane. In membrane-compromised cells fluoresce strongly upon binding to intracellular nucleic acids.

For SG uptake assay, overnight cultures of *Candida* (ATCC 10231) were harvested by centrifugation, washed three times with HEPES buffer and resuspended in the same buffer. The cells were incubated with 1 μM SG in the dark prior to the addition of peptide. The increase in emission intensity at 520 nm was monitored after addition of various concentrations of peptide (λex = 485 nm). 1% Triton-X100 was added at the end of the experiment to determine the % uptake.

*Results:* The ability of B4010 to permeabilise the cytoplasmic membrane was assessed by SYTOX Green uptake assay. The fluorogenic dye stains cells that have compromised plasma membrane and displayed strong fluorescence upon binding to intracellular nucleic acid. A marked and rapid increase in the fluorescence of *C. albicans* upon addition of B4010 was observed confirming the membranolytic action (Figure 5B).

Addition of B4010 to *C. albicans* incubated with 1 μM SG resulted in rapid uptake of the dye with a concomitant increase in fluorescence intensity in a concentration-dependent manner (Figure 5B). At ½× MIC (13%) and 1× MIC values (24%), a weak permeation was observed. About 70% cells were membrane compromised after the addition of B4010 at 2× MIC in 10 mins. However, a higher level of dye uptake (>90%) was observed within 25 mins when the concentration of B4010 was increased to 4× MIC.

*diS-C3-5 membrane depolarisation*

Change in membrane potential of *C. albicans* upon addition of B4010 was monitored by the release of potential sensitive probe diS-C3-5 dye. Briefly, 1 ml of overnight grown mid-log phase Candida cells in 5 mM HEPES buffer (pH 7.0) was mixed with 10 μM diS-C3-5 dye and incubated at 37 °C for 1-2 h in a thermo shaker. 800 μL of the dye-loaded cell suspension was transferred to a stirred quartz cuvette and the change in fluorescence intensity was monitored at an emission wavelength ($\lambda_{em}$) of 670 nm (excitation wavelength, 622 nm) using a Quanta Master spectrofluorimeter (Photon Technology International, NJ, USA). The excitation and emission bandwidths were set at 1 and 2 nm, respectively. Once a constant fluorescence level was achieved, 10 μL of concentrated peptide solution in HEPES buffer was added so that the final concentration of peptide was 0.22 - 22 μM. The change in fluorescence intensity was monitored continuously for 1 or 2 h. To study the effects of energy poisons and ion-channel inhibitors, the additives were added prior to the addition of 5.5 μM B4010. The change in fluorescence intensity was monitored as before. Since yeasts maintain a negative resting membrane potential inside the cell, in order to determine if the candidacidal action of B4010 was due to dissipation of electrochemical gradients across the membrane, using a potential sensitive probe, diS-C3-5 was used. Figure 5C shows the concentration-dependent dissipation of membrane potential upon addition of B4010 using *C .albicans* loaded with the potential sensitive probe. The time course of depolarization occurred instantaneously upon addition of B4010, indicating release of probe from the cells. However, addition of peptide 2× above the MIC had no significant influence on the final membrane potential dissipation.

To verify if the dissipation of membrane potential is linked to candidacidal activity, a cell viability assay was performed under identical conditions. A complete loss of viability was observed when the concentration of peptide exceeded 4× MIC. Together with the SG uptake assay, these results confirmed that B4010 caused rapid membrane potential dissipation and permeabilizes the cytoplasmic membrane of *C. albicans* in a concentration-dependent manner.

The extracellular release of metal ions and ATP upon challenging *C. albicans* with B4010 was assessed to determine if perturbation of the membrane would affect its barrier function.

*Measurement of extracellular cations*

Overnight cultured late logarithmic phase *C. albicans* was harvested by centrifugation, washed 5 times with 10 mM HEPES (pH 7.0), resuspended in the same buffer and adjusted to an OD600 = 0.4. To 5 ml of this suspension, B4010 (final concentration 5.5 μM) was added and incubated at 37 °C for 2 h. The mixture was centrifuged at 3,000 g and the presence of $K^+$, $Ca^{2+}$ and $Mg^{2+}$ in the supernatant was estimated by Perkin Elmer Dual-view Optima 5300 DV inductively coupled plasma - optical emission spectrometry (ICP-OCS Massachusetts, USA) available at CMMAC facilities (Department of Chemistry, National University of Singapore).

*ATP bioluminescence assay*

The extracellular ATP levels upon challenging *C. albicans* with B4010 were determined as reported before (Koshlukova *et al.,* 1999). Cells (OD600 ≈ 0.6) were incubated for 1.5 h at 37 °C with or without various additives for 2 hours at 37 °C with orbital shaking. B4010 (5.5 μM) was added and incubated for another 1.5 h at 37 °C with shaking. Each tube was then centrifuged at 5000 g for 5 mins. Then 225 μL of boiling TE buffer (50 mM Tris, 2 mM EDTA, pH 7.8) was added to the 25 μl of the supernatant and mixed well. This mixture was boiled again for another 2 minutes and stored at 4°C until further examination. 100 μL of a luciferin-luciferase ATP assay mixture was added to 100 μL of the supernatant and luminescence was monitored using the Infinite M200 microplate reader (Tecan Group Ltd., Switzerland). For the time-course experiment, cells (OD600 = 0.4) were treated with 5.5 μM peptide for various time intervals. For dose-dependent studies, the concentration of the peptide was varied from 0.4 - 44 μM. The extracellular ATP concentration was determined from calibration curve obtained by ATP assay kit (Molecular Probes, OR, USA) as per the manufacturer's instruction.

*Results:* The background $K^+$ and $Ca^{2+}$ concentration in the supernatant were 43.4 ± 2 μM and 2.5 ± 0.5 μM, respectively. After incubation of *C. albicans* with B4010 (5.5 μM) for 2 h, more than two fold elevation of potassium (104 ± 4.2 μM) and calcium (5.8 ±1.3 μM) concentration was observed, whereas no significant changes in the levels of $Na^+$ and $Mg^{2+}$ ions were observed. ATP bioluminescence assay indicated a rapid release of ATP from *C. albicans* upon addition of B4010 (Figure 5D inset). The release of ATP was also dependent on the concentration of peptide with a maximum around 4× MIC of B4010 (Figure 5D). orphological changes in *C. albicans* treated with B4010 were investigated by scanning electron microscopy. Untreated cells displayed round, dome-shaped morphologies with a smooth surface (Figure 5E). However, the cell surface of *C. albicans* incubated with B4010 for 30 mins revealed severe damage with bud scars appearing on the surface (Figure 5F). In certain cells release of irregular material was also visible (Figure 5F *inset*), suggesting membrane-lytic action of B4010.

*Candidacidal activity of B4010 in presence of energy poisons and ion channel inhibitors*

For the effects of energy poisons and ion channel inhibitors, yeast cells ($10^5$-$10^6$ CFU/ml) were incubated for 2 h with or without additives at 37 °C. A final concentration of 5.5 μM B4010 peptide was added to the cell suspension and incubated further for another 1.5 h at 37 °C. Each cell suspension was further diluted and 100 μl of the cells were plated and incubated at 37 °C. SDA plates with additives (and no peptide) served as the positive control. Cell viability was obtained by counting the number of colonies formed in each plate after 48 h incubation at 37 °C. The final concentrations of the additives were: CCCP (5 μM), NaN3 (5 mM), 4-AP (1 mM), NPPB (0.5 mM), Gadolinium II chloride (1 mM) and tetra ethyl ammonium chloride (15 mM). The reported values were averages of two independent duplicate experiments. Control experiments with all the additives were also performed to assess the toxicity of the additives to *C. albicans.*

*Results:*

To study the effect of metabolic activity of *C. albicans* on the susceptibility to B4010, the diS-C3-5 loaded cells were incubated with 5 μM CCCP (an uncoupler of proton gradients) or 5 mM $NaN_3$ (which blocks both classical and alternative pathways of mitochondrial inhibition) and the changes in fluorescence intensity upon addition of B4010 were monitored. Addition of CCCP resulted in a strong reduction in fluorescence intensity indicating collapse of the membrane potential. Subsequent addition of B4010 had weak changes in the transmembrane potential (Figure 6A). As shown before by Veerman et al., addition of $NaN_3$ to dye-loaded cells caused weak depolarization. Subsequent addition of B4010 resulted in an increase in fluorescence intensity (Figure 6A). However, ~25 fold re+duction in fluorescence intensity was observed in the azide-treated cells compared to control cells. Removal of $NaN_3$ by centrifugation reversed the intensity change, indicating that the energy poison inhibits the candidacidal activity reversibly. These results suggested that both proton uncoupler and energy poison significantly decreased the B4010-induced depolarization of the cytoplasmic membrane. *C. albicans* cells pre-incubated with CCCP conferred partial protection from B4010-induced lethality whereas a complete protection was observed in the presence of $NaN_3$ (Figure 6B).

The effect of CCCP/$NaN_3$ on ATP release by B4010 was also investigated. Consistent with the cell viability assays, ATP bioluminescence assay indicated significant increase in the extracellular ATP levels in B4010-treated cells (Figure 6C). However, cells pretreated with CCCP/$NaN_3$ followed by the addition of B4010 resulted in significant decrease in ATP release compared to B4010 treated cells (Figure 6C).

*Effects of ion-channel inhibitors on antifungal activity of B4010:* It was observed that B4010 caused rapid release of $K^+$ as well as ATP and that the addition of external K+ decreased the extent of cell death with > 16-fold increase in MIC at elevated ion concentrations prompted the question whether ion-channel inhibitors could protect *C. albicans* from B4010. The effects of non-specific organic cationic inhibitors (TEA and 4-AP) as well as yeast stretch-activated ion channel blocker $Gd^{3+}$ on candidacidal activity of B4010 were tested. The peptide caused significant loss of viability in cells that were pretreated with TEA and 4-AP (Figure 6B). Consistent with the loss of viability, a similar amount of ATP was released from the cells pretreated with TEA and 4-AP after B4010 addition as was observed without these inhibitors (Figure 6C). On the other hand, cells incubated with $Gd^{3+}$ provided partial protection (54 ± 6% loss of viability) against B4010-induced killing. Addition of B4010 to *C. albicans* cells pretreated with the anion channel inhibitor, NPPB, conferred significant protection (12 ± 4% killing) of C. albicans from B4010 (Figure 6B). ATP release assays further confirmed the absence or reduced ATP efflux from additives that conferred complete or partial protection, respectively (Figure 6C).

To determine if the loss of B4010 activity was associated with alterations in the membrane potential, the effect of additives for a change in intensity of diS-C3-5 dye loaded cells before and after the addition of B4010 was monitored. TEA or 4-AP did not alter the membrane potential whereas subsequent addition of B4010 resulted in complete loss of membrane potential and the magnitude of intensity changes was similar to the one observed in cells without prior addition to 4-AP or TEA (Figure 6D). When $Gd^{3+}$ was added to dye-loaded *C. albicans,* a significant depolarization was observed. Further addition of B4010 caused weak dissipation of membrane potential, augmenting the cell viability and ATP release assays. Cells incubated with NPPB resulted in collapse of membrane potential without affecting the cell viability. Further addition B4010 to NPPB-treated cells prevented dissipation of membrane potential (Figure 6D) and abrogated the candidacidal properties of B4010. The dye release assays in the presence of various additives suggested that a resting negative membrane potential and metabolic activity are crucial for candidacidal activity of B4010.

Small unilamellar vesicles (SUVs) were prepared using PC/PE/PI or PS lipids (5:2.5:2.5) containing 15 wt% ergosterol or PC/Cholesterol (10:1) (Makovitzki *et al.,* 2006). For the preparation of PC/PE/PI or PS/Erg SUV, the lipids were dissolved in chloroform/methanol (2:1, v/v) in a glass tube. Ergosterol was dissolved in same solvent and added to lipid mixture to make final ergosterol content of 15 wt%. The Lipid-ergosterol mixture was dried using nitrogen gas to form lipid layer. The film was hydrated in a buffer containing 20mM PBS (pH 7), vortexed and sonicated. Each cycle of sonication (5 sec, 40 °C) is followed by freezing in liquid nitrogen and thawing on a water bath kept at 37 °C. The procedure was repeated 5-6 times until an optically clear dispersion appeared. The SUV was divided into two parts. To

the first part 50 mM calcein was added and incubated for 1 h. The excess calcein was removed by injecting 100 μL of the calcein-loaded SUV's into a Ultrahydrogel™ 250 (7.8 mm × 300 mm) column equilibrated with 20mM PBS (pH 7.0) and eluted isochratically at a flow rate of 1 ml/min for 1.5 column volumes. The calcein-loaded liposomes were mixed with calcein-free liposome (1:1) to adjust final liposome concentration. Peptide concentration was added to obtain 1:30 or 1:15 peptide:liposome ratio. Changes in the fluorescence signal after addition of peptide or Triton X-100 was measured on a PTI spectrofluorometer, using an excitation wavelength of 480 nm and emission wavelength of 512 nm. The Percentage calcein released at each time point was calculated using the equation:

$$\%\text{Calcein Release} = \frac{A_0 - A_{min}}{A_0 - A_{min}} \times 100\%$$

Where A0 is the observed fluorescence intensity upon addition of peptides, Amin is the average intensity at the baseline (before peptide addition), Amax is the average intensity at the saturation phase after adding 1% Triton X-100. Similar protocol was followed for the preparation of PC/Cholesterol SUVs.

*Results:* Figure 7A shows the rapid release of calcein from SUVs when the peptide:lipid ratio was 1:30 and 1:15. Increasing the peptide concentration increased the amount of calcein released. B4010 resulted in about 68% calcein release whereas the scrambled peptide released about 41%, consistent with the higher MIC values observed for the latter peptide. However, no calcein release was observed for natamycin, which complexes ergosterol and does not form large transmembrane pore (Figure 7A). On a mammalian model membrane (PC:Cholesterol), B4010 induced less calcein leakage (Figure 7B) suggesting increased selectivity for the fungal model membrane.

Discussion

In tropical countries, mycoses have high incidence and are responsible for major health and economic problems. The expanding populations of immuno compromised patients, large-scale use of antifungals in food and increased use of medical devices and implants have further raised the incidence of fungal infections. Systemic Candida species account for 40% mortality rates and is the 4th leading cause of hospital acquired blood stream infections in the US. Examples of current antifungals and their properties are shown in Table 7. However, the problem of increase in antifungal resistance and limited choice of antifungal remains.

Table 7 Properties of current antifungals

| Antifungals | Solubility( mg/L) | logP | $T_{1/2}$ | Activity |
|---|---|---|---|---|
| Nystatin | 360 (24°C) | 0.5 | 6h | Broad spectrum |
| Amphotericin B | Insoluble at pH 6/7 | 2.4 | >15 days | Broad spectrum Excellent agains Aspergilus & Candida |
| Natamycin | 4100 (21 °C) | 1.1 | - | Broad spectrum |
| Fluconazole | 1 | 0.4 | 20-50h | Broad spectrum. Less effective against *C. albicans, C. glabrata & C. krusei* |
| Itraconazole | $4.7\times10^{-4}$ | 6.5 | 21 h | Broadspectrum |
| Miconazole | 0.01 (20°C) | 6.1 | 20-25 h | Broad spectrum |
| Ketaconazole | 0.09(25°C) | 4 | 2 h | Broad spectrum |
| Terbinafine | $7.4\times10^{4}$ | 5.9 | 36 h | Broad spectrum |
| 5-FC | $10.5\times10^{5}$ | -1.1 | 2.4-4.8 h | Not active against filamentous fungii |
| Caspofungin | 370 | -2.8 | 9-11 h | Effective against *Candida spp* |

This study reports that multimerisation of a weak antifungal peptide resulted in highly enhanced antifungal and membrane permeabilising activities compared to its monomeric counterpart. The special properties of the B-series of peptides are:

1. Kills many forms of fungus and yeast rapidly (less than 1 hour).
2. Able to kill mutant forms of yeast with changed sterol membrane composition.

3. Resistant to degradation.
4. Resistant to physiological concentrations of monovalent and divalent ions.

The most active peptide which carried 4 copies (B4010) displayed excellent antifungal activities against *Candida* and *Fusarium* strains. Scrambling the sequence resulted in a 2-4 fold decrease in antifungal activity, indicating the importance of amino acid sequence.

Kinetics of candidacidal activity for B4010 was compared with polyene antifungals. For both the ATCC and clinical isolates, B4010 caused 3 log reductions of viable cells in less than 1 h at 2x or 4x MIC values whereas the polyene antifungals require an elevated concentration and longer time to achieve similar end points. The antifungal properties of B4010 were assessed in the presence of monovalent and divalent cations as well as in complex biological fluids. The results showed that the antifungal activity of B4010 was not altered by physiologically relevant ionic strengths although at high concentration of $K^+$ ions a reduced activity was observed. The strong depolarizing activity of $K^+$ on yeast cells at elevated concentration may be responsible for the partial loss of antifungal activity.

When incubated with trypsin or tear fluid for 6 h, B4010 retained significant antifungal potency. These results suggest improved stability of B4010 in complex biological environments. In the presence of human serum, the MIC values of the peptide against two clinical isolates were elevated by about 16 fold. It is likely that the interaction of B4010 with albumin or other proteins may be responsible for the increased MIC values. Taken together these results highlight considerable antifungal efficacy of B4010 in complex biological fluids.

Several mechanisms have been documented in the evolution of resistance to azole and polyene antifungals by *C. albicans.* Qualitative or quantitative changes in the sterol structure and composition by altering the specific steps in the ergosterol biosynthetic pathways are amongst the most important mechanisms of azole and polyene resistance known. Analysis of the sterol composition in azole-resistant *C. albicans* strains from AIDS and leukemia patients indicated the accumulation of ergosta-7,22-dienol. It has been shown that mutations in *ERG4, ERG6* and *ERG3* of *S. cerevisiae* displayed enhanced resistance to polyene and azole antifungals. However, all the yeast mutants which carry altered sterol structure and composition are hyper sensitive to B4010. It is interesting to note that *erg3Δ* mutants that are intrinsically resistant to azoles and polyene antifungals are more susceptible to B4010 than are the erg2*D* and *erg2D6D* mutants. It is possible that the enhanced membrane fluidity resulting from sterol alterations may contribute to increased permeability of B4010, thus leading to the observed hyper susceptibility of the mutant strains.

The therapeutic potential of host defense peptides is also limited by increased cell toxicity and haemolytic activity. In comparison to natamycin and amphotericin B, the peptide was non-haemolytic to rabbit erythrocytes at higher concentration. However, the cytotoxicity of the peptide to HCE cells was comparable to natamycin and superior to that of amphotericin B. B4010 did not affect the corneal reepithelialization rate in rabbit nor displayed acute toxicity in mice indicating safety of the peptide in surgical settings.

Since many antifungal peptides require an intact cell wall to exert antifungal action, the affinity of B4010 for cell wall polysaccharides was examined. B4010 had no affinity for b-D-glucan or chitin as no coprecipitation was observed in the pull-down experiments. To probe the membrane targeting properties and to correlate the rapid candidacidal activity, the effect of concentration of B4010 on the kinetics of membrane permeabilization was monitored by SG uptake and diS-C3-5 release assays. At all concentrations, a rapid SG uptake was observed although the maximum uptake was achieved at 4× the MIC. These results support the time-kill kinetics assays that complete killing occurred at 4× MIC in 30 minutes. diS-C3-5 dye release studies suggested a weak dissipation of the membrane potential at lower concentrations and maximum dissipation was observed above 2× MIC. A loss of viability in yeast cells exposed to 4× MIC of B4010 under identical conditions suggests that dissipation of membrane potential is linked to candidacidal activity. The rapid dissipation of membrane potential and SG uptake may indicate direct interaction of the peptide with the cytoplasmic membrane.

Challenging *C. albicans* with B4010 caused 2 fold increase in $K^+$- and $Ca^{2+}$-ions indicating membrane damage. The extracellular release of ATP from yeast cells treated with B4010 is concentration-dependent with maximum efflux at 4x MIC. The kinetics of ATP efflux at 4x MIC reached a maximum within 30 minutes, in accordance with kinetics of candidacidal activity. SEM studies showed that C. *albicans* treated with B4010 displayed rough with disrupted morphologies and extensive blebbing, again pointing to the observations that membrane is the principal and presumably the critical target for the peptide.

This study showed that additives which alter the cytoplasmic membrane potential or energy metabolism confer substantial protection of *C. albicans* from B4010 induced lethality. At 5 μM, CCCP has been shown to cause depolarization of the cytoplasmic membrane whereas a high concentration (>50 μM) is required for mitochondrial depolarization. Cell viability assays confirmed that cells pretreated with 5 μM CCCP provided partial protection (45% viable cells) from B4010. diS-C3-5 assay indicated collapse of membrane potential in the presence of 5 μM CCCP. A similar effect was observed in the presence of anion channel inhibitor, DIDS. These results suggest that the alterations in transnegative electrochemical gradient may potentially be the underlying mechanism of protection of *C. albicans* from B4010. It has been shown that the membrane potential of *S. cerevisiae* is -76±5 mV whereas for *C. albicans* the value is -120 mV

(55,56). The reduced membrane potential of *S. cerevisiae* may account for the observed higher MIC values *S. cerevisiae* (5.5 μM) compared to *C. albicans* (0.37-1.4 μM).

In support of this, a high concentration of extracellular $K^+$ strongly depolarises the yeast cells without affecting cell viability and subsequent addition of B4010 caused little changes in the membrane potential and partial protection (40% viable cells) from B4010. Furthermore, ion-channel inhibitors, which did not alter the membrane potential, failed to protect yeast cells from B4010. However, the presence of $NaN_3$ completely abrogated the candidacidal activity of B4010. It has been shown that $NaN_3$ alters the fluidity of the plasma membrane of yeast without affecting the membrane potential. Consistent with this, addition of membrane fluidizer rescued the antifungal activity of B4010. Therefore, this suggests that the candidacidal action of B4010 is linked to plasma membrane potential and fluidity of the membrane.

The effect of B4010 on calcein-loaded SUVs containing purified lipids and ergosterol point unambiguously towards membrane-lytic action of the peptide. The results from these studies further indicated that tetrabranching and the aminoacid sequence are important for membrane disruption since a decreased calcein release was observed in the presence of B2088 and Sc_B4010 peptides. In cholesterol-containing zwitterionic SUVs, however, B4010 caused reduced calcein release suggesting weaker interactions with mammalian model membrane. A high % of calcein release and retention of extended conformation of the peptide in mixed liposome containing ergosterol supported the results obtained from the MD simulation results. In addition, the low MIC against yeasts and high EC50 values against HCE cells confirm that B4010 selectively damages the fungal membrane.

In conclusion, this study showed that assembling 4 copies of a weakly active peptide on a branched lysine core amplifies the properties and overcomes several limitations of the linear antimicrobial peptides. In addition, the peptide is hyper potent against several yeast strains with altered sterol structure and composition, thus suggesting their potential to combat resistance. The peptide is non-toxic when tested in vitro and in vivo. B4010 targets the cytoplasmic membrane and caused rapid dissipation of membrane potential and loss of intracellular components. Additives that alter the membrane potential or metabolic activity have profound influence on the antifungal properties. Using SUVs, this study showed that the peptide was more selective to fungal model membrane than for the mammalian model membrane. The first arginine residue of the putative sequence played an important role in mediating the interactions with the negatively charged fungal model membrane. In support of this, replacement of the first arginine resulted in ~2-4 fold decrease in antifungal activity. Although it is difficult to simulate the *in vivo* conditions, this study combines extensive experimental and computer simulation studies to advance understanding of the interaction of antifungal peptides with model lipids and provide guidance for the rational design of therapeutically important new antifungal drug with the promise of combating resistance.

References

Crosson C.E., Klyce S.D. and Beuerman R.W. (1986) Epithelial wound closure in the rabbit cornea. A biphasic process. Invest Ophthalmol Vis Sci. 27:464-73.

Koshlukova, S. E., Lloyd, T. L., Araujo, M. W. and Edgorton, M. (1999) Salivary histatin 5 induces non-lytic release of ATP from Candida albicans leading to cell death. J. Biol. Chem. 274: 8872-18879.

Makovitzki, A., Avrahami, D. and Shai, Y. (2006) Ultrashort antibacterial and antifungal lipopeptides. Proc Natl Acad Sci USA. 103:15997-6002.

Oren, Z. and Shai, Y. (1997) Selective lysis of bacteria but not mammalian cells by diastereomers of melittin: structure-function study. Biochemistry 36:1826-1835.

Veerman, E. C., Valentijn-Benz, M., Nazmi, K., Ruissen, A. L., Walgreen-Weterings E., Van Marie, J., Doust, A. B., Van't Hof, W., Bolscher, J. G. and Amerongen, A. V. (2007) Energy depletion protects Candida albicans against antimicrobial peptides by rigidifying its cell membrane. J Biol Chem. 282:18831-41.

SEQUENCE LISTING

<110> Singapore Health Services Pte Ltd Agency for Science, Technology and Research

<120> Tetrabranched peptides and anlogues as a new class of antimicrobials and tehir preparation thereof

<130> FP6608

<150> SG 201206715-3
<151> 2012-09-07

<160> 28

<170> PatentIn version 3.5

<210> 1
<211> 8
<212> DNA
<213> artificial

<220>
<223> initial peptide monomer

<400> 1
rgrkvvrr 8

<210> 2
<211> 8
<212> DNA
<213> artificial

<220>
<223> 1st R of initial peptide monomer replaced with A

<400> 2
agrkvvrr 8

<210> 3
<211> 8
<212> DNA
<213> artificial

<220>
<223> 2nd G of initial peptide monomer replaced with A

<400> 3
rarkvvrr 8

<210> 4
<211> 8
<212> DNA
<213> artificial

<220>
<223> 3rd R of initial peptide monomer replaced with A

<400> 4
rgakvvrr 8

<210> 5
<211> 8
<212> DNA
<213> artificial

<220>
<223> 4th K of initial peptide monomer replaced with A

<400> 5
rgravvrr 8

<210> 6
<211> 8
<212> DNA
<213> artificial

<220>
<223> 5th v of initial peptide monomer replaced with A

<400> 6
rgrkvarr 8

<210> 7
<211> 8
<212> DNA
<213> artificial

<220>
<223> 6th V of of initial peptide monomer replaced with A

<400> 7
rgrkvarr 8

<210> 8
<211> 8
<212> DNA
<213> artificial

<220>
<223> 7th A of of initial peptide monomer replaced with A

<400> 8
rgrkvvar 8

<210> 9
<211> 8
<212> DNA
<213> artificial

<220>
<223> 8th R of of initial peptide monomer replaced with A

<400> 9
rgrkvvra 8

<210> 10
<211> 8
<212> DNA
<213> artificial

<220>
<223> 3rd R and 4th K of initial peptide monomer replaced with As

<400> 10
rgaavvrr 8

<210> 11
<211> 8
<212> DNA
<213> artificial

<220>
<223> 7th A and 8th Rs of initial peptide monomer replaced with As

<400> 11
rgrkvvaa 8

<210> 12
<211> 8
<212> DNA
<213> artificial

<220>
<223> 3rd R and 5th v of initial peptide monomer replaced with As

<400> 12
rgakavrr 8

<210> 13
<211> 8
<212> DNA
<213> artificial

<220>
<223> 5th and 6th vs of initial peptide monomer replaced with As

<400> 13
rgrkaarr 8

<210> 14
<211> 8
<212> DNA
<213> artificial

<220>
<223> 3rd R, 4th K and 5th v of initial peptide monomer replaced with As

<400> 14
rgaaavrr 8

<210> 15
<211> 8
<212> DNA
<213> artificial

<220>
<223> 3rd r, 5th and 6th vs of initial peptide monomer replaced with As

<400> 15
rgakaarr 8

<210> 16
<211> 8
<212> DNA
<213> artificial

<220>
<223> 4th K, 5th and 6th V of initial peptide monomer replaced with As

<400> 16
rgraaarr 8

<210> 17
<211> 8
<212> DNA
<213> artificial

<220>
<223> 3rd R, 4th K, 5th and 6th vs of initial peptide monomer replaced with As

<400> 17
rgaaaarr 8

<210> 18
<211> 8
<212> DNA
<213> artificial

<220>
<223> 5th and 6th vs; 7th and 8th Rs of initial peptide monomer replaced with As

<400> 18
rgrkaaaa 8

<210> 19
<211> 8
<212> DNA
<213> artificial

<220>
<223> scrambled initial peptide monomer

<400> 19
vrgrvrkr 8

<210> 20
<211> 7
<212> DNA
<213> artificial

<220>
<223> R of initial peptide monomer deleted

<400> 20
grkvvrr 7

<210> 21
<211> 6
<212> DNA

<213> artificial

<220>
<223> RG of initial peptide monomer deleted

<400> 21
rkvvrr 6

<210> 22
<211> 5
<212> DNA
<213> artificial

<220>
<223> RGR of initial peptide monomer deleted

<400> 22
kvvrr 5

<210> 23
<211> 4
<212> DNA
<213> artificial

<220>
<223> RGRK of initial peptide monomer deleted

<400> 23
vvrr 4

<210> 24
<211> 3
<212> DNA
<213> artificial

<220>
<223> RGRKV of of initial peptide monomer deleted

<400> 24
vrr 3

<210> 25
<211> 2
<212> DNA
<213> artificial

<220>
<223> RGRKVV of initial peptide monomer deleted

<400> 25
rr 2

<210> 26
<211> 1
<212> DNA
<213> artificial

<220>

<223> RGRKVVR of initial peptide monomer deleted

<400> 26
r 1

<210> 27
<211> 10
<212> DNA
<213> artificial

<220>
<223> 10 bp linear monomer

<400> 27
rgrkvvrrkk 10

<210> 28
<211> 19
<212> DNA
<213> artificial

<220>
<223> linear retrodimer

<400> 28
rgrkvvrrkk krrvvkrgr 19

## Claims

1. An isolated peptide tetramer selected from the group consisting of $[(RGAAVVRR)_2K]_2KK_i$, $[(RGRKVVAA)_2K]_2KK_i$, $[(RGAKAVRR)_2K]_2KK_i$, $[(RGAAAVRR)_2K]_2KK_i$, $[(RGAKAARR)_2K]_2KK_i$, $[(RGRAAARR)_2K]_2KK_i$, $[(RGAAAARR)_2K]_2KK_i$, $[(RGRKAAAA)_2K]_2KK_i$; wherein i = 0 or 1.

2. The isolated peptide tetramer according to claim 1, wherein the isolated peptide tetramer consists of $[(RGAAAARR)_2K]_2KK_i$.

3. The isolated peptide tetramer according to claim 1 or 2, wherein i = 1.

4. An isolated peptide tetramer according to any one of claims 1 to 3 for use as a medicament, pharmaceutical composition and/or antimicrobial composition and/or in therapy.

5. An *in vitro* method of inhibiting and/or reducing the growth of at least one microorganism comprising contacting the microorganism with at least one isolated peptide tetramer according to any one of claims 1 to 3.

6. A contact lens and/or eye drop solution, a pharmaceutical and/or antimicrobial composition, a composition for coating a device and/or a kit comprising an isolated peptide tetramer according to any one of claims 1 to 3.

7. A method of preparing the isolated peptide tetramer according to any one of claims 1 to 3.

8. The method according to claim 7,

(A) wherein the peptide tetramer comprises four identical peptide monomers, comprising the steps of:

(i) providing at least one solid phase;
(ii) coupling a first protected K residue to the solid phase;
(iii) removing a protective group from one amine group in the first K residue;
(iv) linking a second protected K residue to the first K residue;

(v) removing a protective group from one amine group in the second K residue;
(vi) linking two protected K residues to the second K residue;
(vii) removing the protected groups from the two linked K residues;
(viiii) providing additional chain extension by linking protected amino acid residues; according to the sequence of the respective peptide monomer from the C-terminus to the N-terminus, wherein after each linking, the protecting groups are moved for the next linking;
(ix) terminating the linking of amino acid residues depending on the number of residues to be added; and
(x) optionally, releasing the multimer from the solid phase;

(B) wherein the peptide tetramer comprises four identical peptide monomers, comprising the steps of:

(i) providing at least one solid phase;
(ii) coupling a first protected K residue to the solid phase;
(iii) removing a protective group from one amine group in the first K residue;
(iv) linking a second protected K residue to the first K residue;
(v) removing a protective group from one amine group in the second K residue;
(vi) linking two protected K residues to the second K residue;
(vii) removing the protected groups from the two linked K residues;
(viii) linking one peptide monomer with a protected terminal amine group to each amine group from the two K residues; and
(iv) optionally, releasing the multimer from the solid phase;

(C) wherein the peptide tetramer comprises four identical peptide monomers, comprising the steps of:

(i) providing a least one solid phase;
(ii) coupling a first K residue to the solid phase;
(iii) linking two protected K residues to the coupled first K residue;
(iv) removing the protective groups from the linked K residues;
(v) providing additional chain extension by linking protected amino acid residues, according to the sequence of the respective peptide monomer from the C-terminus to the N-terminus, wherein after each linking, the protecting groups are removed for the next linking;
(vi) terminating the linking of amino acid residues depending on the number of residues to be added; and
(vii) optionally, releasing the multimer from the solid phase.

or (D) wherein the peptide tetramer comprises four identical peptide monomers, comprising the steps of:

(i) providing a least one solid phase;
(ii) coupling a first K residue to the solid phase;
(iii) linking two protected K residues to the coupled first K residue;
(iv) removing the protective groups from the linked K residues;
(v) linking one peptide monomer with a protected terminal amine group to each amine group from the two K residues; and
(vi) optionally, releasing the multimer from the solid phase.

## Patentansprüche

1. Isoliertes Peptid-Tetramer, ausgewählt aus der Gruppe bestehend aus $[(RGAAVVRR)_2K]\ _2KK_i$, $[(RGRKVVAA)_2K]\ _2KK_i$, $[(RGAKAVRR)_2K]_2KK_i$, $[(RGAAAVRR)_2K]_2KK_i$, $[(RGAKAARR)_2K]_2KK_i$, $[(RGRAAARR)_2K]_2KK_i$, $[(RGAAAARR)_2K]_2KK_i$, $[(RGRKAAAA)_2K]_2KK_i$; wobei i = 0 oder 1.

2. Isoliertes Peptid-Tetramer nach Anspruch 1, wobei das isolierte Peptid-Tetramer aus $[(RGAAAARR)_2K]_2KK_i$, besteht.

3. Isoliertes Peptid-Tetramer nach Anspruch 1 oder 2, wobei i = 1.

4. Isoliertes Peptid-Tetramer nach einem der Ansprüche 1 bis 3 zur Verwendung als Medikament, pharmazeutische Zusammensetzung und/oder antimikrobielle Zusammensetzung und/oder bei einer Therapie.

**5.** *In-vitro*-Verfahren zum Hemmen und/oder Reduzieren des Wachstums mindestens eines Mikroorganismus, umfassend das Kontaktieren des Mikroorganismus mit mindestens einem isolierten Peptid-Tetramer nach einem der Ansprüche 1 bis 3.

**6.** Kontaktlinsen- und/oder Augentropfenlösung, pharmazeutische und/oder antimikrobielle Zusammensetzung, Zusammensetzung zum Beschichten einer Vorrichtung und/oder eines Kits, die ein isoliertes Peptid-Tetramer nach einem der Ansprüche 1 bis 3 umfasst.

**7.** Verfahren zum Herstellen des isolierten Peptid-Tetramers nach einem der Ansprüche 1 bis 3.

**8.** Verfahren nach Anspruch 7,

(A) wobei das Peptid-Tetramer vier identische Peptid-Monomere umfasst, wobei das Verfahren die folgenden Schritte umfasst:

(i) Bereitstellen mindestens einer Festphase;
(ii) Koppeln eines ersten geschützten K-Rests an die Festphase;
(iii) Entfernen einer Schutzgruppe von einer Aminogruppe im ersten K-Rest;
(iv) Verbinden eines zweiten geschützten K-Rests mit dem ersten K-Rest;
(v) Entfernen einer Schutzgruppe von einer Aminogruppe im zweiten K-Rest;
(vi) Verbinden zweier geschützter K-Reste mit dem zweiten K-Rest;
(vii) Entfernen der Schutzgruppen von den zwei verbunden K-Resten;
(viiii) Bereitstellen einer zusätzlichen Kettenverlängerung durch Verbinden von geschützten Aminosäureresten gemäß der Sequenz des entsprechenden Peptid-Monomers vom C-Ende bis zum N-Ende, wobei jeweils nach dem Verbinden die Schutzgruppen für das nächste Verbinden entfernt werden;
(ix) Beenden des Verbindens von Aminosäureresten je nach Anzahl der Reste, die hinzugefügt werden soll; und
(x) optional Lösen des Multimers von der Festphase;

(B) wobei das Peptid-Tetramer vier identische Peptid-Monomere umfasst, wobei das Verfahren die folgenden Schritte umfasst:

(i) Bereitstellen mindestens einer Festphase;
(ii) Koppeln eines ersten geschützten K-Rests an die Festphase;
(iii) Entfernen einer Schutzgruppe von einer Aminogruppe im ersten K-Rest;
(iv) Verbinden eines zweiten geschützten K-Rests mit dem ersten K-Rest;
(v) Entfernen einer Schutzgruppe von einer Aminogruppe im zweiten K-Rest;
(vi) Verbunden zweier geschützter K-Reste mit dem zweiten K-Rest;
(vii) Entfernen der Schutzgruppen von den zwei verbundenen K-Resten;
(viii) Verbinden jeweils eines Peptid-Monomers mit einer geschützten endständigen Aminogruppe mit jeder Aminogruppe von den zwei K-Resten; und
(iv) optional Lösen des Multimers von der Festphase;

(C) wobei das Peptid-Tetramer vier identische Peptid-Monomere umfasst, wobei das Verfahren die folgenden Schritte umfasst:

(i) Bereitstellen mindestens einer Festphase;
(ii) Koppeln eines ersten K-Rests an die Festphase;
(iii) Verbinden zweier geschützter K-Reste mit dem gekoppelten K-Rest;
(iv) Entfernen der Schutzgruppen von den verbundenen K-Resten;
(v) Bereitstellen einer zusätzlichen Kettenverlängerung durch Verbinden von geschützten Aminosäureresten gemäß der Sequenz des entsprechenden Peptid-Monomers vom C-Ende bis zum N-Ende, wobei jeweils nach dem Verbinden die Schutzgruppen für das nächste Verbinden entfernt werden;
(vi) Beenden des Verbindens von Aminosäureresten je nach Anzahl der Reste, die hinzugefügt werden soll; und
(vii) optional Lösen des Multimers von der Festphase

oder (D) wobei das Peptid-Tetramer vier identische Peptid-Monomere umfasst, wobei das Verfahren die folgenden Schritte umfasst:

(i) Bereitstellen mindestens einer Festphase;
(ii) Koppeln eines ersten K-Rests an die Festphase;
(iii) Verbinden zweier geschützter K-Reste mit dem gekoppelten K-Rest;
(iv) Entfernen der Schutzgruppen von den verbundenen K-Resten;
(v) Verbinden jeweils eines Peptid-Monomers mit einer geschützten endständigen Aminogruppe mit jeder Aminogruppe von den zwei K-Resten; und
(vi) optional Lösen des Multimers von der Festphase.

**Revendications**

**1.** Tétramère de peptide isolé choisi dans le groupe constitué de $[(RGAAVVRR)_2K]\ _2KK_i$, $[(RGRKVVAA)_2K]\ _2KK_i$, $[(RGAKAVRR)_2K]\ _2KK_i$, $[(RGAAAVRR)_2K]\ _2KK_i$, $[(RGAKAARR)_2K]\ _2KK_i$, $[(RGRAAARR)_2K]\ _2KK_i$, $[(RGAAAARR)_2K]\ _2KK_i$, $[(RGRKAAAA)_2K]_2KK_i$ ; dans lequel i = 0 ou 1

**2.** Tétramère de peptide isolé selon la revendication 1, dans lequel le tétramère de peptide isolé est constitué de $[(RGAAAARR)_2K]_2KK_i$.

**3.** Tétramère de peptide isolé selon la revendication 1 ou 2, dans lequel i = 1.

**4.** Tétramère de peptide isolé selon l'une quelconque des revendications 1 à 3 pour une utilisation en tant que médicament, composition pharmaceutique et/ou composition antimicrobienne et/ou en thérapie.

**5.** Procédé *in vitro* d'inhibition et/ou de réduction de la croissance d'au moins un micro-organisme comprenant la mise en contact du micro-organisme avec au moins un tétramère de peptide isolé selon l'une quelconque des revendications 1 à 3.

**6.** Lentille de contact et/ou solution ophtalmique, composition pharmaceutique et/ou antimicrobienne, composition pour l'enrobage d'un dispositif et/ou kit comprenant un tétramère de peptide isolé selon l'une quelconque des revendications 1 à 3.

**7.** Procédé de préparation du tétramère de peptide isolé selon l'une quelconque des revendications 1 à 3.

**8.** Procédé selon la revendication 7,

(A) dans lequel le tétramère de peptide comprend quatre monomères de peptide identiques, comprenant les étapes de :

(i) la fourniture d'au moins une phase solide ;
(ii) le couplage d'un premier résidu K protégé à la phase solide ;
(iii) l'élimination d'un groupe protecteur à partir d'un groupe amine dans le premier résidu K ;
(iv) la liaison d'un second résidu K protégé au premier résidu K ;
(v) l'élimination d'un groupe protecteur à partir d'un groupe amine dans le second résidu K ;
(vi) la liaison de deux résidus K protégés au second résidu K ;
(vii) l'élimination des groupes protégés à partir des deux résidus K liés ;
(viiii) la fourniture d'une extension de chaîne supplémentaire par la liaison de résidus d'acides aminés protégés ; selon la séquence du monomère de peptide respectif de l'extrémité C-terminale à l'extrémité N-terminale, dans lequel après chaque liaison, les groupes protecteurs sont déplacés pour la liaison suivante ;
(ix) la terminaison de la liaison des résidus d'acides aminés selon le nombre de résidus à ajouter ; et
(x) éventuellement, la libération du multimère à partir de la phase solide ;

(B) dans lequel le tétramère de peptide comprend quatre monomères de peptide identiques, comprenant les étapes de :

(i) la fourniture d'au moins une phase solide :
(ii) le couplage d'un premier résidu K protégé à la phase solide ;

(iii) l'élimination d'un groupe protecteur à partir d'un groupe amine dans le premier résidu K ;
(iv) la liaison d'un second résidu K protégé au premier résidu K ;
(v) l'élimination d'un groupe protecteur à partir d'un groupe amine dans le second résidu K ;
(vi) la liaison de deux résidus K protégés au second résidu K ;
(vii) l'élimination des groupes protégés à partir des deux résidus K liés ;
(viii) la liaison d'un monomère de peptide avec un groupe amine terminal protégé à chaque groupe amine provenant des deux résidus K ; et
(iv) éventuellement, la libération du multimère à partir de la phase solide ;

(C) dans lequel le tétramère de peptide comprend quatre monomères de peptide identiques, comprenant les étapes de :

(i) la fourniture d'au moins une phase solide ;
(ii) le couplage d'un premier résidu K à la phase solide ;
(iii) la liaison de deux résidus K protégés au premier résidu K couplé ;
(iv) l'élimination des groupes protecteurs à partir des résidus K liés ;
(v) la fourniture d'une extension de chaîne supplémentaire par la liaison de résidus d'acides aminés protégés, selon la séquence du monomère de peptide respectif de l'extrémité C-terminale à l'extrémité N-terminale, dans lequel après chaque liaison, les groupes protecteurs sont éliminés pour la liaison suivante ;
(vi) la terminaison de la liaison des résidus d'acides aminés selon le nombre de résidus à ajouter ; et
(vii) éventuellement, la libération du multimère à partir de la phase solide,

ou (D) dans lequel le tétramère de peptide comprend quatre monomères de peptide identiques, comprenant les étapes de :

(i) la fourniture d'au moins une phase solide ;
(ii) le couplage d'un premier résidu K à la phase solide ;
(iii) la liaison de deux résidus K protégés au premier résidu K couplé ;
(iv) l'élimination des groupes protecteurs à partir des résidus K liés ;
(v) la liaison d'un monomère de peptide avec un groupe amine terminal protégé à chaque groupe amine provenant des deux résidus K ; et
(vi) éventuellement, la libération du multimère à partir de la phase solide.

Monomer

RGRKVVRRKK

B2088

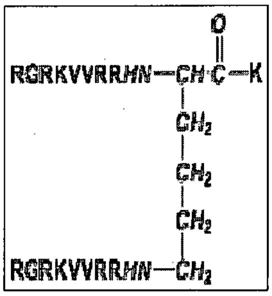

B4010

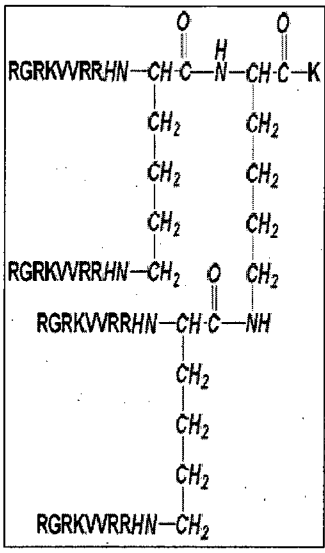

Scrambled B4010

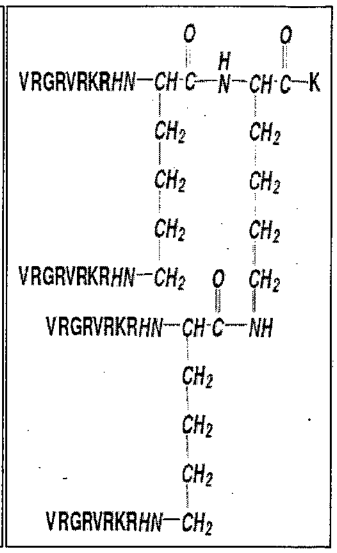

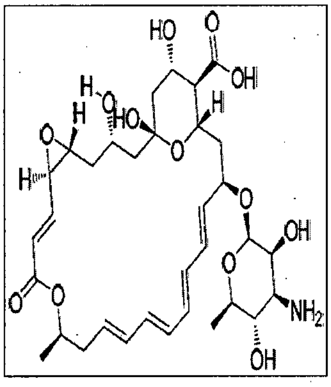

Natamycin

Linear Retodimer

RGRKVVRRKKKRRVVKRGR

Figure 1

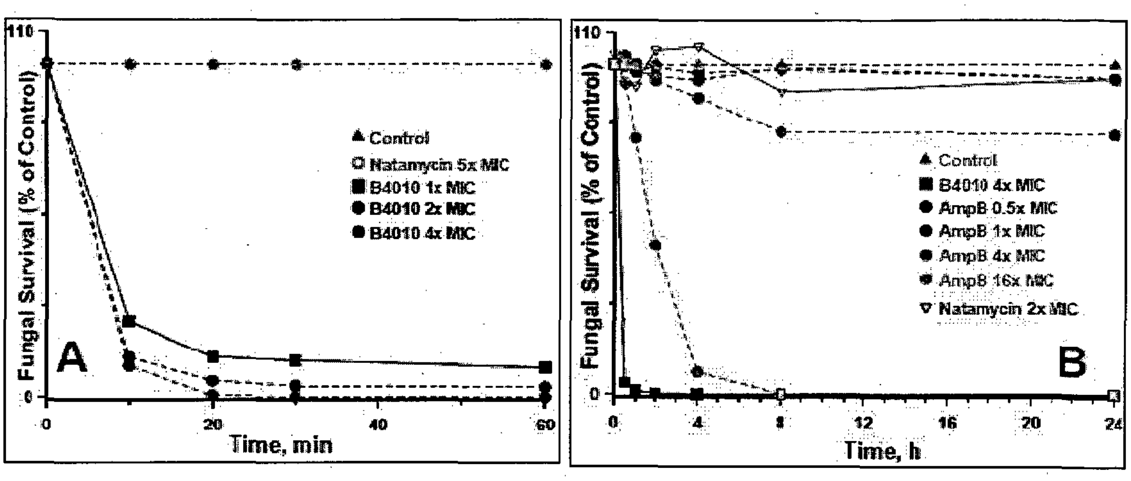


Figure 2

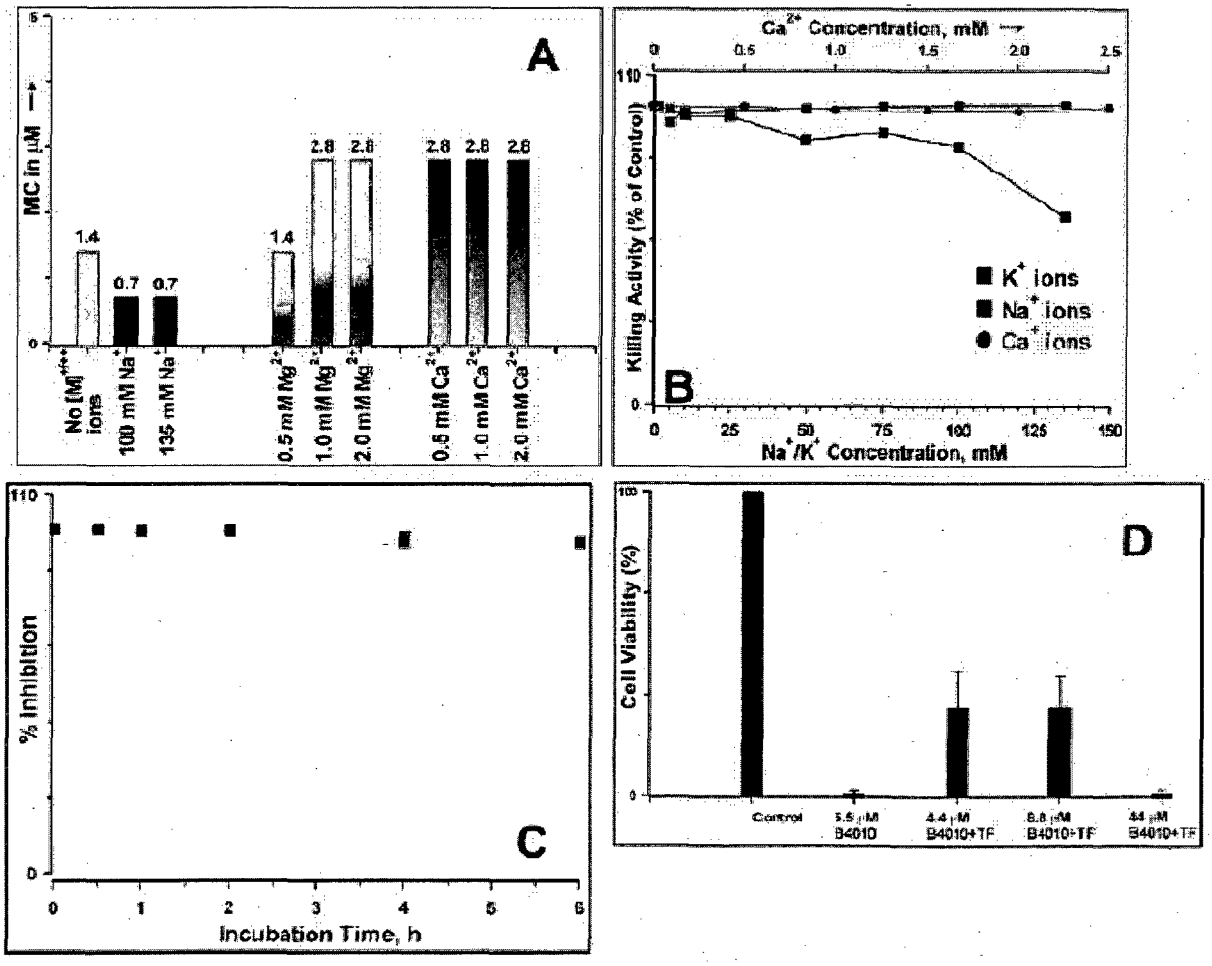


Figure 3

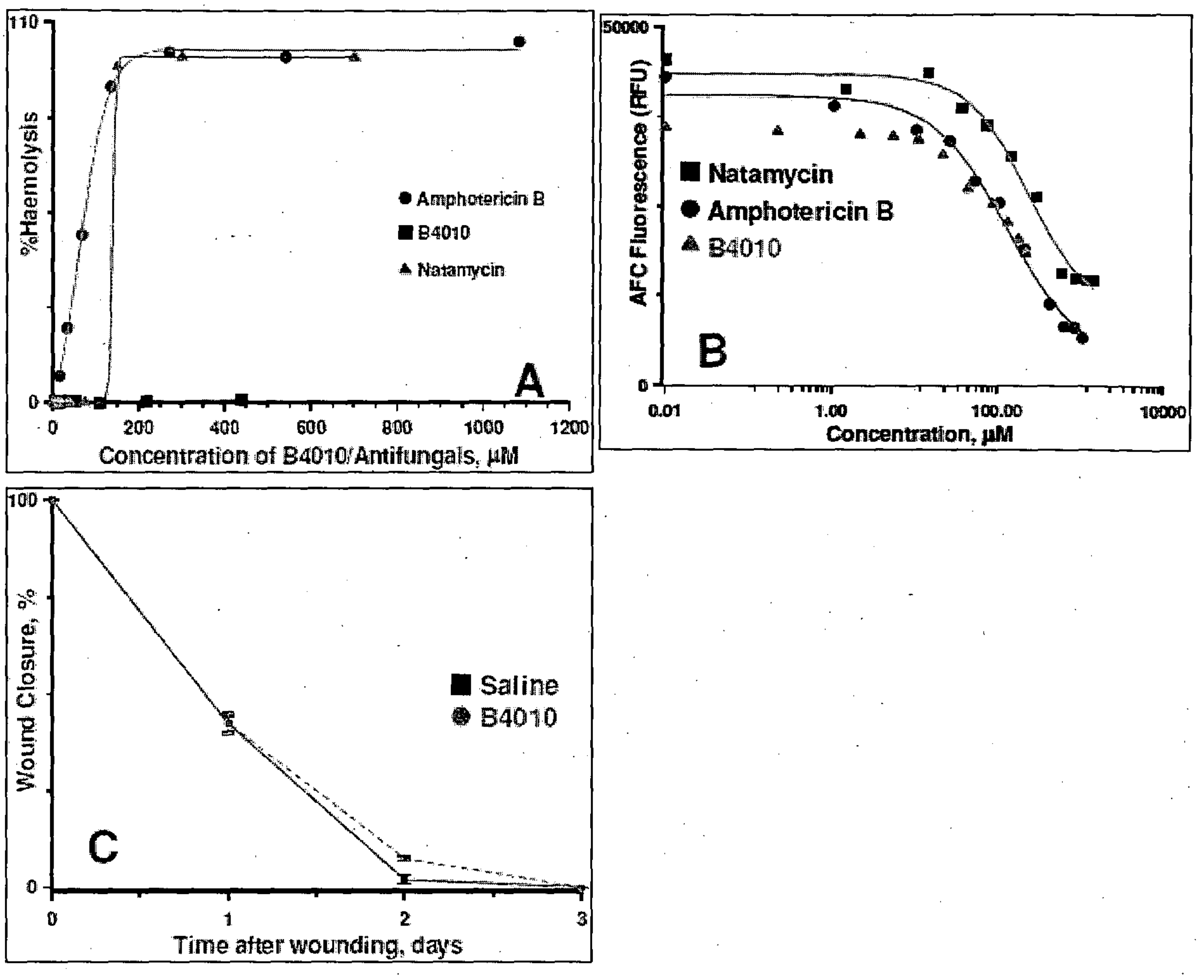
110
%Haemolysis
0
0
200
400
600
800
1000
1200
Concentration of B4010/Antifungals, µM
Amphotericin B
B4010
Natamycin
A
50000
AFC Fluorescence (RFU)
0
0.01
1.00
100.00
10000
Concentration, µM
Natamycin
Amphotericin B
B4010
B
100
Wound Closure, %
0
0
1
2
3
Time after wounding, days
Saline
B4010
C

Figure 4

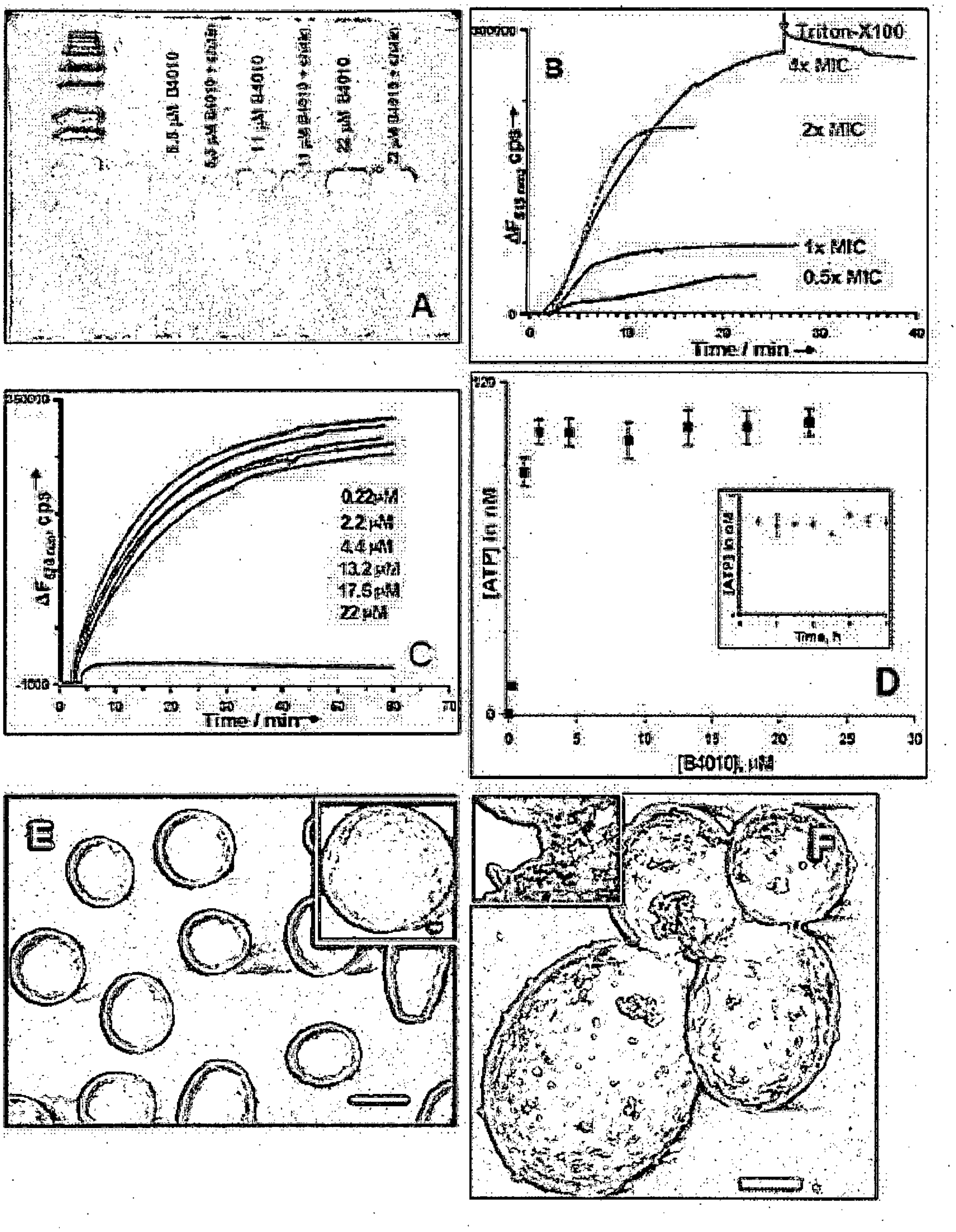
5.5 µM B4010
11 µM B4010
22 µM B4010
A
Triton-X100
4x MIC
2x MIC
1x MIC
0.5x MIC
B
Time / min
0.22 µM
2.2 µM
4.4 µM
13.2 µM
17.5 µM
22 µM
C
Time / min
[ATP] in nM
D
[B4010], µM
E
F

Figure 5

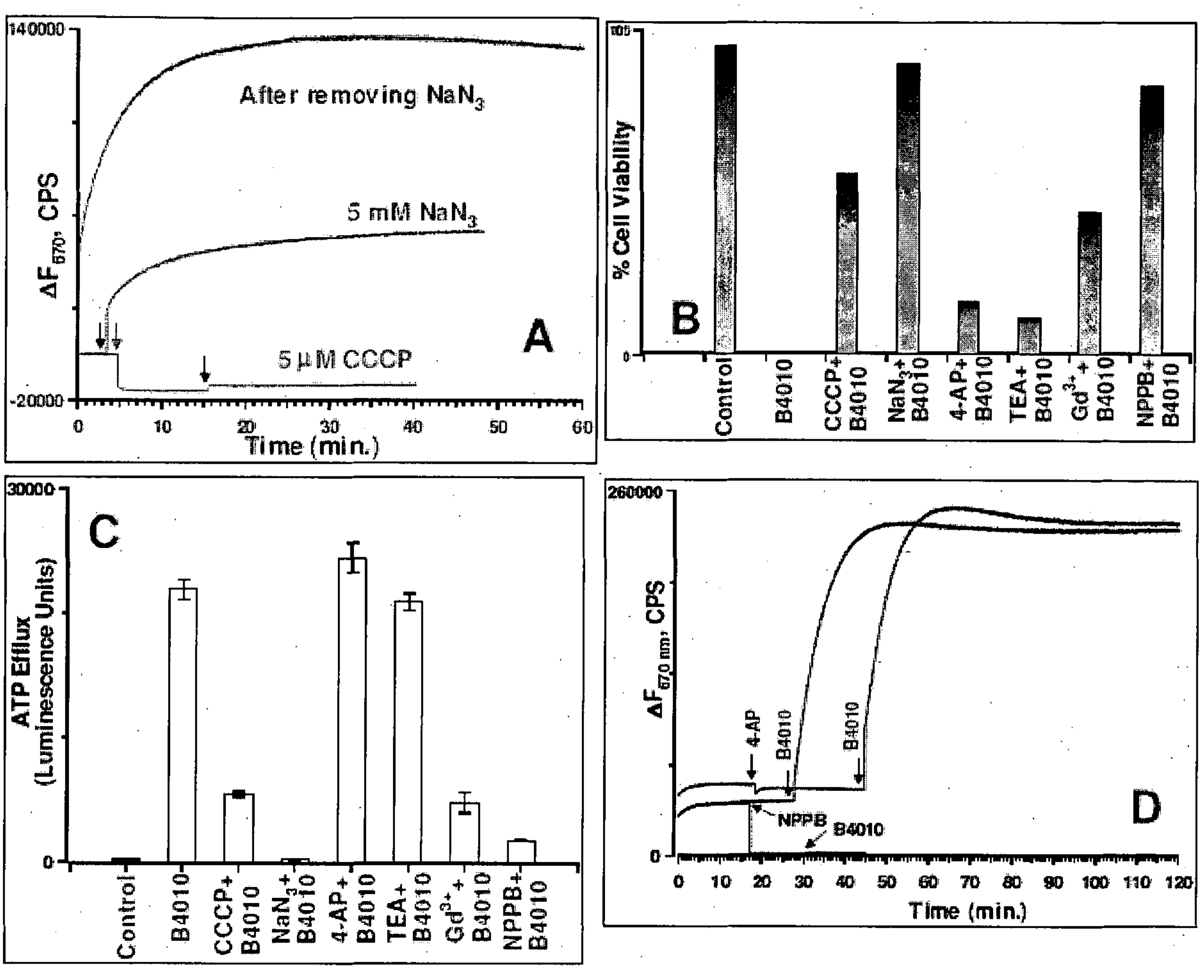
140000
-20000
ΔF670, CPS
After removing NaN3
5 mM NaN3
5 μM CCCP
A
0 10 20 30 40 50 60
Time (min.)
% Cell Viability
B
Control
B4010
CCCP+ B4010
NaN3+ B4010
4-AP+ B4010
TEA+ B4010
Gd3+ + B4010
NPPB+ B4010
30000
0
ATP Efflux (Luminescence Units)
C
260000
ΔF670 nm, CPS
4-AP
B4010
NPPB
D
0 10 20 30 40 50 60 70 80 90 100 110 120
Time (min.)

Figure 6

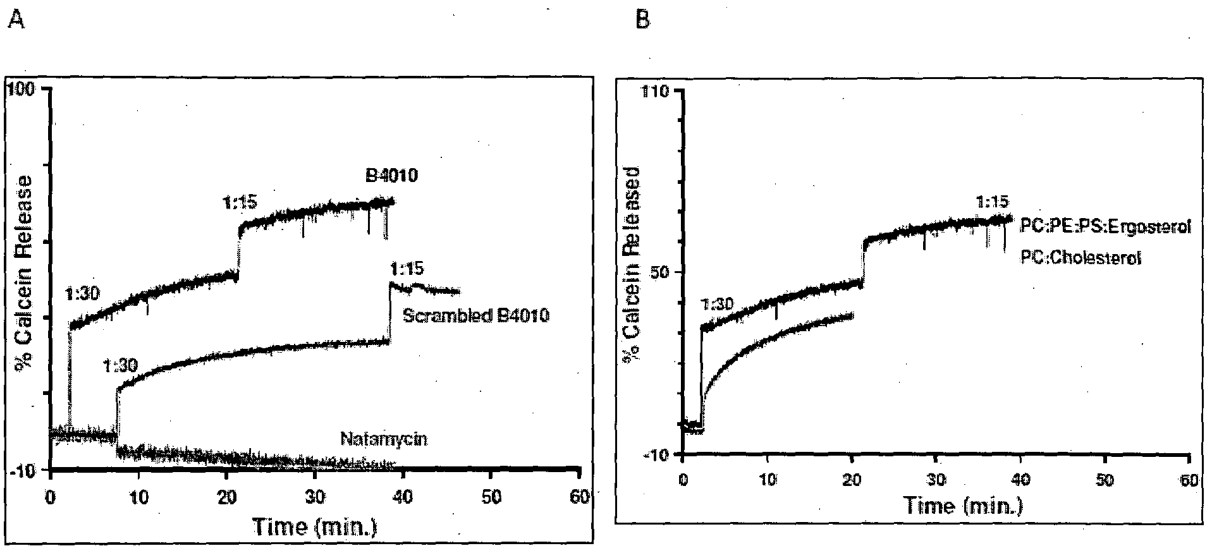


Figure 7

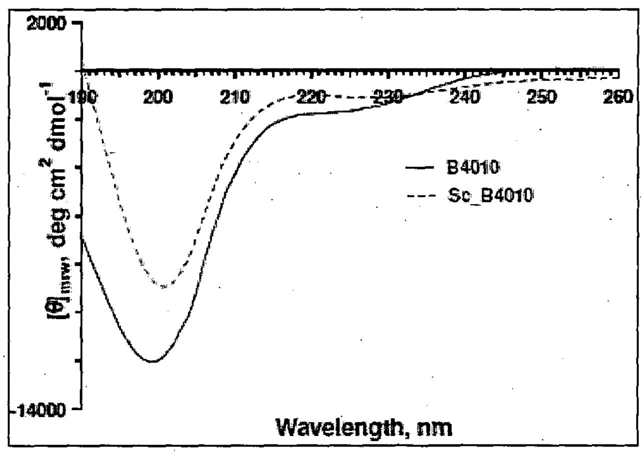


Figure 8

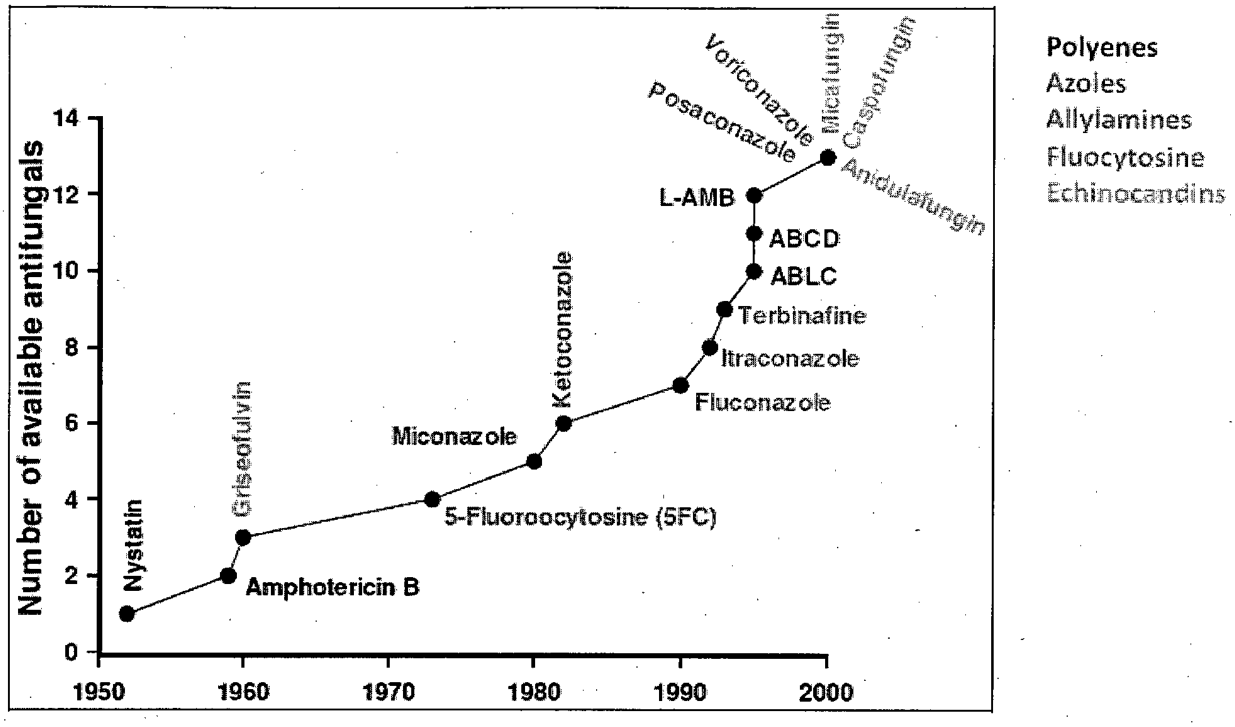
Number of available antifungals
0
2
4
6
8
10
12
14
1950
1960
1970
1980
1990
2000
Nystatin
Amphotericin B
Griseofulvin
5-Fluoroocytosine (5FC)
Miconazole
Ketoconazole
Fluconazole
Itraconazole
Terbinafine
ABLC
ABCD
L-AMB
Posaconazole
Voriconazole
Micafungin
Caspofungin
Anidulafungin
Polyenes
Azoles
Allylamines
Fluocytosine
Echinocandins

Figure 9

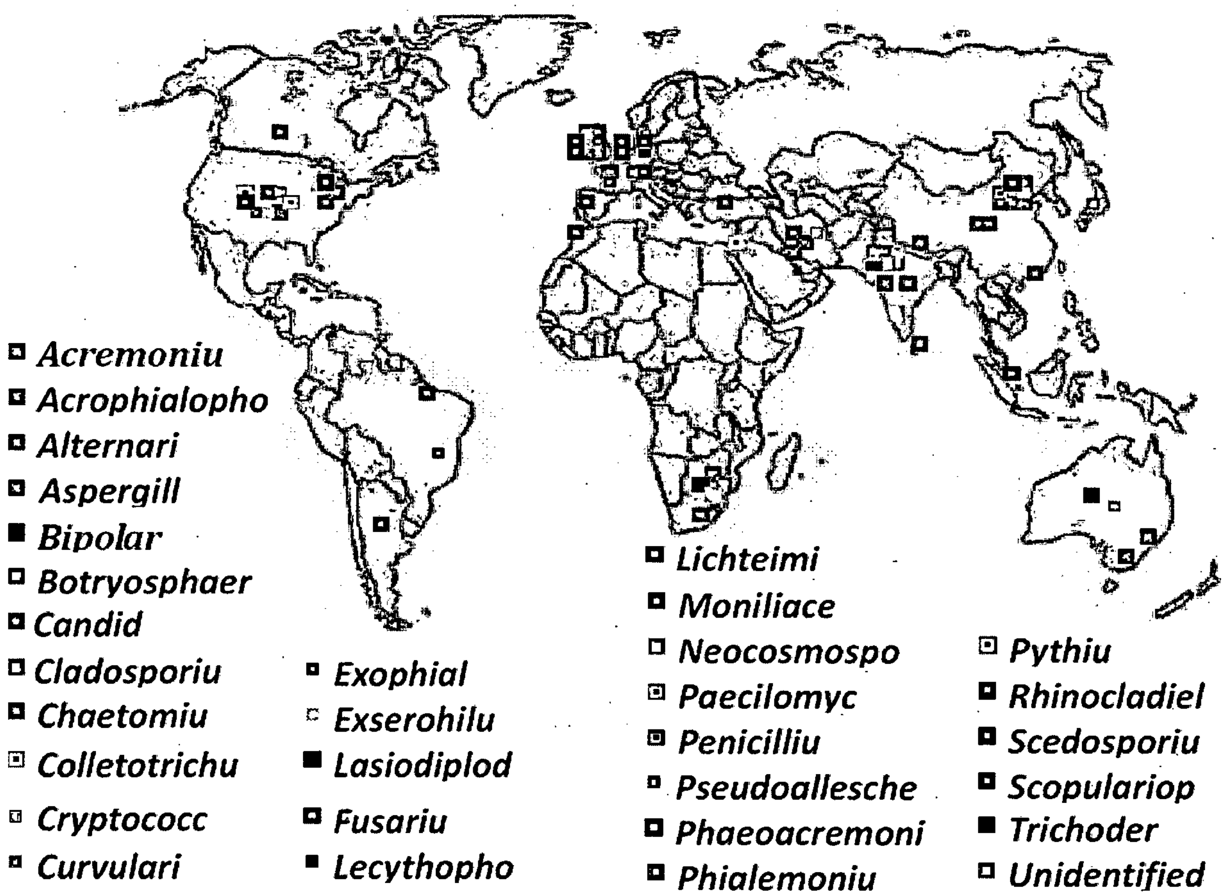
Acremoniu
Acrophialopho
Alternari
Aspergill
Bipolar
Botryosphaer
Candid
Cladosporiu
Chaetomiu
Colletotrichu
Cryptococc
Curvulari
Exophial
Exserohilu
Lasiodiplod
Fusariu
Lecythopho
Lichteimi
Moniliace
Neocosmospo
Paecilomyc
Penicilliu
Pseudoallesche
Phaeoacremoni
Phialemoniu
Pythiu
Rhinocladiel
Scedosporiu
Scopulariop
Trichoder
Unidentified

Figure 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009131548 A1
- SG 2012067153

### Non-patent literature cited in the description


- **ZHOU et al.** *Amino Acids,* 2011, vol. 40, 123-133
- **LIU et al.** *Int. J. Pept. Res. Ther.,* 2010, vol. 16, 199-213
- **TAN et al.** *J. Biomed. Mat. Res. B,* 2012, vol. 100B, 2090-2100
- **GREEN.** Molecular Cloning: A Laboratory Manual. Cold Springs Harbor Laboratory, 2012
- **CROSSON C.E. ; KLYCE S.D. ; BEUERMAN R.W.** Epithelial wound closure in the rabbit cornea. A biphasic process. *Invest Ophthalmol Vis Sci.,* 1986, vol. 27, 464-73
- **KOSHLUKOVA, S. E. ; LLOYD, T. L. ; ARAUJO, M. W. ; EDGORTON, M.** Salivary histatin 5 induces non-lytic release of ATP from Candida albicans leading to cell death. *J. Biol. Chem.,* 1999, vol. 274, 8872-18879
- **MAKOVITZKI, A. ; AVRAHAMI, D. ; SHAI, Y.** Ultrashort antibacterial and antifungal lipopeptides. *Proc Natl Acad Sci USA.,* 2006, vol. 103, 15997-6002
- **OREN, Z. ; SHAI, Y.** Selective lysis of bacteria but not mammalian cells by diastereomers of melittin: structure-function study. *Biochemistry,* 1997, vol. 36, 1826-1835
- **VEERMAN, E. C. ; VALENTIJN-BENZ, M. ; NAZMI, K. ; RUISSEN, A. L. ; WALGREEN-WETERINGS E. ; VAN MARIE, J. ; DOUST, A. B. ; VAN'T HOF, W. ; BOLSCHER, J. G. ; AMERONGEN, A. V.** Energy depletion protects Candida albicans against antimicrobial peptides by rigidifying its cell membrane. *J Biol Chem.,* 2007, vol. 282, 18831-41